# EUROPEAN PATENT APPLICATION

(11) **EP 3 208 262 A1**
(43) Date of publication of application: **23.08.2017**
(21) Application number: 15851060.2
(22) Date of filing: 15.10.2015
(51) Int. Cl.: C07D 209/30, C07D 209/32, C07D 209/40, C07D 209/80, C07D 209/82, A61K 31/4045, A61K 31/403, A61P 25/02, A61P 25/14, A61P 25/16, A61P 25/28

(54) **COMPOUNDS DERIVED FROM 3-ALKYLAMINO-1H-INDOLE ACRYLATE, AND THE USE THEREOF IN THE TREATMENT OF NEURODEGENERATIVE DISEASES**

(30) Priority: 15.10.2014 ES 201400810
(71) Applicant: Fundación Para La Investigación Biomédica Del Hospital De La Princesa, 28006 Madrid (ES); Universidad Autónoma de Madrid, 28049 Madrid (ES); DNS Neuroscience, 28050 Madrid (ES)
(72) Inventor: LEON MARTINEZ, Rafael, 28029 Madrid (ES); BUENDIA ABAITUA, Izaskun, 28028 Madrid (ES); NAVARRO GONZALEZ DE MESA, Ellsa, 28004 Madrid (ES); MICHALSKA, Patrycja, 28514 Nuevo Baztán (ES); GAMEIRO ROS, Isabel, 28034 Madrid (ES); LÓPEZ VIVO, Alicia, 28045 Madrid (ES); EGEA MAIQUEZ, Javier, 28026 Madrid (ES); GARCIA LOPEZ, Manuela, 28029 Madrid (ES); GARCIA GARCIA, Antonio, 28430 Alpedrete (ES)
(74) Representative: ZBM Patents - Zea, Barlocci & Markvardsen
(86) International application number: PCT/ES2015/000139
(87) International publication number: WO 2016/059269

(57) **Abstract**

The invention relates to the methods for producing derivatives of 3-alkylamino-1H-indole acrylate (I) with transcription factor Nrf2-inducing activity, free radical scavenging activity and neuroprotective ability. The invention also relates to the use of the derivatives according to the invention for the treatment of diseases, the pathogenesis of which involves oxidative stress, or diseases involving the deregulation of the activity of phase II genes activated by the factor Nrf2, such as Alzheimer's disease, Parkinson's disease, Huntington's disease, multiple sclerosis, ictus or amyotrophic lateral sclerosis.

## Description

### Technical Field

The present invention focuses in the pharmaceutical sector with application in the prevention and/or treatment of diseases and any other affection mediated by oxidative stress, specifically in the identification of chemical compounds useful for the treatment of neurodegenerative diseases with cognitive or motor decline secondary to the neuronal neurodegeneration, such as Alzheimer's disease (AD), Parkinson's disease (PD), amyotrophic lateral sclerosis (ALS) and Huntington's disease (HD) or stroke, as well as in other conditions with neuronal loss as a consequence of autoimmune diseases such as multiple sclerosis (MS).

### Background Art

Neurodegenerative diseases will be the most important health challenge of this generation, if no drug is found to delay disease progression, the socioeconomic situation may become unsustainable within the next 30 years. The world health organization has alerted about the progress of these diseases and has established them as priority worldwide. Nowadays, it is estimated that the number of patients that currently suffer a neurodegenerative disease is about 35.6 millions worldwide; this number will duplicate in 2030 and will triplicate in 2050, reaching over 115,4 millions (Organization, 2012: 112). There are common characteristics to all neurodegenerative diseases such as high levels of oxidative stress and low grade chronic neuroinflammation. The majority of these diseases show aberrant protein aggregates, lipid peroxidation, protein nitration and DNA oxidation (Di Carlo y col., 2012, Free Radic Res, 46: 1327-38). Based on these observations, it is believed that oxidative stress, mitochondrial dysfunction and neuroinflammation play a major role in the development of these diseases.

The production of reactive oxygen species (ROS) or free radicals, is progressively increased with aging, being this a major factor in these pathologies. Therefore there has to be a fine regulation to maintain an effective balance between the production and elimination of free radicals. Neurons are endowed with different mechanisms that act as redox sensors to identify and initiate an antioxidant response in order to prevent the accumulation o excessive production of pro-oxidative species. The Antioxidant Response Element (ARE) is a regulation element found adjacent to the 5'DNA which precedes regions that codify for a great number of cytoprotective genes and their expression is regulated in response to oxidative stress (Rushmore y col., 1991, J Biol Chem, 266: 11632-9, Joshi y col., 2012, Recent Pat CNS Drug Discov, 7: 218-29). In the presence of a toxic stimulus or by chemical activation, the transcription factor Nrf2 ("nuclear factor (erythroid-derived 2)-like 2") is released from its co-repressor protein Keap 1 and translocates to the nucleus where it dimerizes with small proteins Maf to produce the activation-trans complex that binds to the ARE sequence (Nguyen y col., 2004, Free Radic Biol Med, 37: 433-41). Consequently, the trans-activation of ARE, induced by Nrf2, coordinates de expression of numerous genes to combat oxidative stress and their toxicity in may tissues and cell types (Ramos-Gomez y col., 2001, Proc Natl Acad Sci U S A, 98: 3410-5, Enomoto y col., 2001, Toxicol Sci, 59: 169-77, Gao y col., 2004, Proc Natl Acad Sci U S A, 101: 10446-51, Lee y col., 2003, J Biol Chem, 278: 37948-56). The Nrf2 antioxidant enzyme systems include redox regulation [superoxide dismutase (SOD), catalase (CAT), sulforedoxin (Srx), thioredoxin (Trx), peroxiredoxin (Prdx)], synthesis and metabolism of glutathione[glutathione peroxidase (Gpx), glutathione reductase (GR), γ-glutamine cysteine ligase (GCL) and synthase (GCS)], recycle of quinones [NAD(P)H quinone oxidoreductase (NQO1)] and iron homeostasis [hemo-oxygenase-1 (HO-1), ferritin].

Besides protecting against the toxicity induced by chemical agents, carcinogenesis or aging (Thimmulappa y col., 2002, Cancer Res, 62: 5196-203, Suh y col., 2004, Proc Natl Acad Sci U S A, 101: 3381-6, Zhang y col., 2004, Mol Cancer Ther, 3: 885-93), the transcription factor Nrf2 directly inhibits apoptosis induced by FAS (a substrate for the protease Caspase-3) and the P-ERK dependent survival effector (Ohtsubo y col., 1999, Cell Death Differ, 6: 865-72, Kotlo y col., 2003, Oncogene, 22: 797-806, Cullinan y col., 2004, J Biol Chem, 279: 20108-17). Depending on the disease, the cellular environment or cellular subtype, certain antioxidant genes are more relevant than others in the brain. Under normal conditions, the expression of Nrf2-ARE dependent genes are less active in neurons that in astrocytes (Lee y col., 2003, J Biol Chem, 278: 12029-38, Kraft y col., 2004, J Neurosci, 24: 1101-12); therefore, neurons depend on astrocytes to protect themselves against oxidative stress (Tanaka y col., 1999, Glia, 28: 85-96).

Deregulation or dysfunction of the Nrf2-ARE pathway has been related to the pathology of different neurodegenerative diseases such as AD, PD, HD and ALS, or to those related to neuronal loss like stroke or autoimmunity like MS. Thereby, the NRf2-ARE pathway has become an important therapeutic target to treat the above mentioned diseases (Joshi y col., 2012, Recent Pat CNS Drug Discov, 7: 218-29) and others.

As to the role of the NRf2-ARE pathway in AD, an increase in the expression of HO-1 in the temporal cortex and hippocampus has been observed in post-mortem brains of these patients when compared to age-matched control subjects (Schipper y col., 2006, Neurobiol Aging, 27: 252-61). Furthermore, enhanced activity of the enzyme NQO1 in astrocytes and neurons has been observed, which indicates high levels of oxidative stress (Wang y col., 2000, Neurobiol Aging, 21: 525-31, Raina y col., 1999, Redox Rep, 4: 23-7). In spite of the high levels of oxidative stress found in AD, in hippocampal neurons these patients, Nrf2 mainly localizes in the cytoplasm (Raina y col., 1999, Redox Rep, 4: 23-7, Ramsey y col., 2007, J Neuropathol Exp Neurol, 66: 75-85). In line with the above mentioned findings, it has reported that induction of endogenous antioxidant pathways causes protection against neuronal toxicity induced by *β*-amyloid peptide (Aβ) (Kanninen y col., 2008, Mol Cell Neurosci, 39: 302-13, Wruck y col., 2008, Mol Pharmacol, 73: 1785-95). These and other *results in vitro and in vivo* indicate that the Nrf2-ARE pathway is a therapeutic target with great potential for the treatment of AD.

As to the role of the Nrf2-ARE pathway in PD, this disease is known to have increased levels of oxidative stress, marked astrogliosis and micorgliosis. In this particular case, although Nr2 localizes in the nucleus, there is no expression of phase II genes (Alam y col., 1997, J Neurochem, 69: 1196-203, Clements y col., 2006, Proc Natl Acad Sci U S A, 103: 15091-6). Furthermore, there are *in vitro* and *in vivo* studies that show that induction of Nrf2 is neuroprotective against toxics that induce Parkinson, as well as in transgenic models of PD (Chen y col., 2009, Proc Natl Acad Sci USA, 106: 2933-8, Innamorato y col., 2010, PLoS One, 5: el1838, Burton y col., 2006, Neurotoxicology, 27: 1094-100, Rojo y col., 2010, Glia, 58: 588-98, Lastres-Becker y col., 2012, Hum Mol Genet, 21: 3173-92).

As to the role of the Nrf2-ARE pathway in HD, this disease is characterized by high levels of oxidative stress and mitochondrial dysfunction as a consequence of alterations in the mitochondrial complexes II, III and IV. In certain models of HD, induction of Nrf2 (by administration of triterpenoids in the food), showed improvement of motor symptoms and increased survival; it also reduced oxidative stress markers and the characteristic atrophy of the striatum. These results suggest that the use of drugs capable of modulating the Nrf2-ARE pathway would be a promising therapeutic strategy to treat HD.

Regarding ALS and the Nrf2-ARE pathway, oxidative stress has been pointed out as one of the major contributors to disease progression. It is worth noting that there are studies that show how overexpression of Nrf2 in astrocytes with hSOD1G93A mutations, completely suppress neuronal toxicity in co-cultures. These observation were also *reproduced in vivo;* when GFAP-Nrf2 mice (mice that overexpress Nrf2) were crossed with different ALS mice models, disease progression was slowed down and survival was increased. Furthermore, treatment with Nrf2 inducers, once ALS symptoms were initiated, also showed significant neuroprotection and slowed down disease progression. Thereby, activation of Nrf2 is a viable therapeutic strategy to treat ALS.

As to the implication of the Nrf2-ARE pathway in MS, this disease is characterized by chronic inflammation of certain nuclei in the central nervous system, loss of myelin that covers neuronal axons (demyelination), axonal loss and neuronal, oligodendrocyte and glial cell death (O'Gorman y col., 2012, Int J Mol Sci, 13: 11718-52). Studies have demonstrated that Nrf2-ARE inducers reduce inflammation (Schimrigk y col., 2006, Eur J Neurol, 13: 604-10, Lee y col., 2012, Int J Mol Sci, 13: 11783-803) and reduce astroglial activation in both *in vitro and in vivo* models (Scannevin y col., 2012, J Pharmacol Exp Ther, 341: 274-84). These compounds also reduce the production of pro-inflammatory cytokines (Lin y col., 2011, ASN Neuro, 3) and the lipopolysaccharide (LPS) pro-inflammatory signal in astrocytes. Treatment with inducers of the Nrf2-ARE pathway is a viable strategy, as shown by the approval by the FDA (Food and Drug Administration) in 2013 of dimethyl fumarate (Tecfidera®) for the treat of MS

In the documents of patents WO2008/136838, WO2008/108825, WO2009/036204, WO2009/146216, WO2010/126605, WO2010/107733, WO2010/036711, WO2010/126605, WO2010/046710, WO2011/0250300, WO2011/156889, WO2012/116362, WO2012/145420, WO2012/149478, WO2013/067036, WO2013/132124, P2013/00667 different options for the treatment of neurodegenerative diseases are proposed based on one o several chemical compounds that induce and/or modulate the Nrf2-ARE pathway as a neuroprotectant and immunomodulation strategy.

### Summary of Invention

The invention refers to the compounds with structure 3-alkylamino-1*H*-indolyl acrylate with Nrf2-ARE pathway inducing capability by releasing the transcription factor Nrf2 from the protein Keap1 and its later translocation to the nucleus with the antioxidant, anti-inflammatory and neuroprotective effects that involves. These compounds bear a new substitution that involves new activities and, besides, the Nrf2 inducing capability depends on those new substituents. In the present invention, it is first described the inclusion of the Nrf2 induction capability combined with the antioxidant capability. These activities resulted from the structural modifications that have led to a new compound. Besides, the compounds object of the present invention possesses the free radical scavenger ability and neuroprotective capability, so they can be potentially useful for the prevention and/or treatment of neurodegenerative diseases. More precisely, they can be useful in the prevention and/or treatment of neurodegenerative diseases that occurs with an increase of oxidative stress and/or a mitochondrial dysfunction. On the other hand, they can also be useful in the treatment of autoimmune diseases that occur with a chronic inflammatory process or cerebral isquemic disease.

These new derivatives present, among others, the following characteristics:
i) Capability of inducing the release of the transcription factor Nrf2,
ii) Free radical scavenging capability,
iii) Neuroprotective capability against different toxic stimuli related to the increase of oxidative stress.

Therefore, in one aspect, the invention refers to a compound of formula (I) (defined later), its salts, prodrugs or solvates. The compound with formula (I) can be used for the treatment of neurodegenerative diseases or autoimmune diseases that occur with inflammation or in brain ischemia diseases.

In another aspect, the invention describes a procedure for obtaining of the mentioned compound of formula (I), its salts, prodrugs or solvates.

In another aspect, the invention includes a pharmaceutical composition that comprises a compound of formula (I), or a salt, a prodrug or a solvate pharmaceutically acceptable thereof.

In another aspect, the invention refers to a pharmaceutical composition that comprises a compound of formula (I), or a salt, a prodrug or a solvate thereof, and a pharmaceutically acceptable vehicle.

In another aspect, the invention describes a process for preparing a compound of formula (I), or a salt, prodrug or solvate thereof.

In another aspect, the invention protects the use of the mentioned compound of formula (I), or its salts, prodrugs or solvates, pharmaceutically acceptable, in the production of a pharmaceutical composition for the prevention or the treatment of neurodegenerative diseases or autoimmune diseases that occur with inflammation, or in brain ischemia diseases.

In the context of the present invention, the following terms have the meaning detailed below:
When the term "independently selected" is used, the substituents to which it refers (for example, groups R, such as groups R₁, R₂, R₃, R₄, R₅, R₆ or R₇ or X or variables such as "n") the groups can be identical or different, or in its case when it is specified.

The term "C₁₋₆ alkyl" refers to a lineal or branched aliphatic chain radical that has between 1 and 6, preferably between 1 and 3 ("C₁₋₃ alkyl), carbon atoms and that is bound to the rest of the molecule by a single bond. This term includes, for example, and in a not limiting sense, methyl, ethyl, n-propyl, i-propyl, n-butyl, t-butyl, n-pentyl, etc.

The term "C₁₋₆ haloalkyl" refers to an alkyl radical as previously defined where, at least one hydrogen atom has been replaced by a halogen atom. This term includes, for example, and in a not limiting sense, fluoromethyl, bromomethyl, iodomethyl, difluoromethyl, trifluoromethyl, 2-fluoroethyl, 2-chloroethyl, 1-fluoroethyl, pentafluoroethyl, 1-fluoropropyl, 2-chloropropyl, 3-fluoropropyl, 3-chloropropyl, 1-fluorobutyl, 1-cholobutyl, 4-fluorobutyl. Preferably, haloalkyl is CF₃.

The term "C₁₋₆ alkoxyl" refers to a -O-alkyl group, where alkyl is as it has been previously defined. Preferably, alkoxyl is methoxyl.

The term "halogen" refers to bromine, chlorine, iodine or fluorine. Preferably, halogen is fluorine, chlorine, or bromine.

The term "cycloalkyl" refers to a mono or polycyclic aliphatic group saturated or partially saturated that has between 3 and 10, preferably between 3 and 6, carbon atoms that is bound to the rest of the molecule by a single bond, including for example, and in a not limiting sense, cyclopropyl, cyclobutyl, cyclohexyl, cyclopentyl, etc.

The term "aryl" refers to an aromatic group that has between 6 and 18, preferably between 6 and 10, carbon atoms, that comprises 1, 2 or 3 aromatic nuclei, bound by a carbon-carbon bond or condensed, including for example and in a not limiting sense phenyl, naphthyl, diphenyl, indenyl, phenanthryl, etc.

The term "heterocycle" refers to a stable ring radical from 3 to 10 members, preferably a ring of 5 or 6 members, that consists of carbon atoms and from one to five heteroatoms selected from the group formed by nitrogen, oxygen and sulphur and that can be partially or totally saturated or that can be aromatic ("heteroaryl"). For the purpose of this invention, the heterocycle can be a monocyclic, bicyclic or tricyclic ring system that can include condensed ring systems. The examples of such heterocycles include, but are not limited to, pyrrolidine, piperidine, piperazine, morpholine, tetrahydrofuran, benzimidazole, benzothiazole, furan, pyrrol, pyridine, pyrimidine, isothiazole, imidazole, indole, purine, quinoline, and thiadiazole.

As it is understood in this technical area, there can be a certain degree of substitution in the previously described radicals. The references of the present document with respect to the substituted groups indicate that the specified radical can be substituted in one or more available positions with one or more substituents. Those substituents include, for example and in a not limiting sense, C₁₋₆ alkyl, C₂₋₆ alkenyl, C₂₋₆ alkynyl, cycloalkyl, aryl, heterocyclic, halogen, CN, NO₂, CF₃, -N(Rₐ)(R_{b}), -OR_{c}, -SR_{d}, -C(O)Rₑ -C(O)OR_{f}, -C(O)N(R_{g})(Rₕ), -OC(O)Rᵢ; in which Rₐ, R_{b}, R_{c}, R_{d}, Rₑ, R_{f}, Rg, Rₕ and Rᵢ are independently selected from hydrogen, alkyl, C₁₋₆, aryl, heterocyclic and trifluoromethyl.

One aspect of the present invention refers to a compound of formula (I): 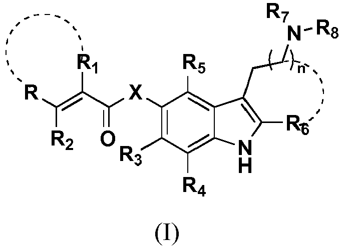 wherein
R is selected from the group consisting of:
   - (C₁-C₆)alkyl optionally substituted by one, two or three halogen atoms selected from fluorine, chlorine, and bromine; (C₃-C₆)cycloalkyl; (C₁-C₆)alkoxyl; (C₃-C₆)cycloalkoxyl; cyano and nitro; or two groups can form together a group -O(CH₂)ₘO-, or -(CH₂)ₚ-, or -CH=CH-CH=CH-; or
   - phenyl optionally substituted by one, two or three groups independently selected from fluorine, chlorine, bromine; (C₁-C₆)alkyl optionally substituted by one, two or three halogen atoms selected from fluorine, chlorine and bromine; (C₃-C₆)cycloalkyl; (C₁-C₆)alkoxyl; (C₃-C₆)cycloalkoxyl; cyano and nitro; or two groups can form together a group -O(CH₂)ₘO-, -(CH₂)ₚ-, or -CH=CH-CH=CH-; and/or
   - an heteroaryl group selected from 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, 3-pyrazinyl, 2-pyrrolyl, 3-pyrrolyl, 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-imidazolyl, 4-imidazolyl, 3-pyrazolyl, 4-pyrazolyl, 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl, 1,3,4-oxadiazol-2-yl, 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl, 1,3,4-thiadiazol-2-yl, 1,2,3-triazol-4-yl, 1,2,4-triazol-3-yl and 5-tetrazolyl, being the heteroaryl group optionally substituted by one, two or three groups independently selected from fluorine, chlorine, and bromine, (C₁-C₆)alkyl, (C₃-C₆)cycloalkyl, (C₁-C₆)alkoxyl, (C₃-C₆)cycloalkoxyl, cyano and nitro;
R₁ and R₂ are selected from the group consisting of hydrogen, (C₁-C₆)alkyl, (C₃-C₆)cycloalkyl, and phenyl, optionally substituted by one, two or three groups selected independently from fluorine, chlorine, and bromine, (C₁-C₆)alkyl, (C₁-C₆)alkoxyl; (C₃-C₆)cycloalkyl, (C₃-C₆)cycloalkoxyl, cyano, nitro and carboxilate; or two groups can form together a group -O(CH₂)_{q}O-, -(CH₂)ᵣ-, or -CH=CH-CH=CH-;
R₃, R₄ and R₅ are selected from the group consisting of hydrogen (C₁-C₆)alkyl, (C₃-C₆)cycloalkyl, and phenyl, optionally substituted by one, two or three groups independently selected from fluorine, chlorine, and bromine, (C₁-C₆)alkyl, (C₁-C₆)alkoxyl; (C₃-C₆)cycloalkyl, (C₃-C₆)cycloalkoxyl, cyano, nitro, and carboxylate; or two groups can form together a group -O(CH₂)qO-, -(CH₂)ᵣ-, or -CH=CH-CH=CH-;
R₆ is selected from the group consisting of hydrogen, (C₁-C₆)alkyl, (C₃-C₆)cycloalkyl, and phenyl, optionally substituted by one, two or three groups independently selected form fluorine, chlorine, bromine, (C₁-C₆)alkyl, (C₁-C₆)alkoxyl; (C₃-C₆)cycloalkyl, (C₃-C₆)cycloalkoxyl, cyano, and nitro;
R₇ is selected from the group consisting of hydrogen, (C₁-C₆)alkyl, (C₃-C₆)cycloalkyl, acetyl or phenyl, optionally substituted by one, two or three groups independently selected from fluorine, chlorine and bromine, (C₁-C₆)alkyl, (C₁-C₆)alkoxyl; (C₃-C₆)cycloalkyl, (C₃-C₆)cycloalkoxyl, cyano and nitro or two groups can form together a group -(CH₂)ₛ-, or -CH=CH-CH=CH-
R₈ is selected from the group consisting of hydrogen, (C₁-C₆)alkyl, (C₃-C₆)cycloalkyl, acetyl or phenyl, optionally substituted by one, two or three groups independently selected from fluorine, chlorine, and bromine, (C₁-C₆)alkyl, (C₁-C₆)alkoxyl; (C₃-C₆)cycloalkyl, (C₃-C₆)cycloalkoxyl, cyano and nitro or two groups can form together a group -(CH₂)ₛ-, or -CH=CH-CH=CH-
   or R₇ = R₈ of formula =C=S;
X is selected from an oxygen atom, a nitrogen atom or a sulphur atom, -SO- or SO₂,
n is an integer selected from 0, 1, 2, 3, 4 or 5;
and their salts, preferably pharmaceutically acceptable salts, pro-drugs or solvates.

The term "pharmaceutically acceptable" refers, preferably, to compositions and molecular entities that are physiologically tolerable and do not produce, normally, an allergic reaction or a similar non-favourable reaction, such as gastric disorders, dizziness and similar, when it is administrated to a human being or animal. The expression "pharmaceutically acceptable" means that it is approved by a regulatory agency such as the European Medicines Agency or the USA regulatory agency, or that it is included in the American Pharmacopoeia or other generally recognized pharmacopoeia for its use in animals and, in a more particular way, in human beings.

The term "salts" as it is used here refers to any salt of the compound with formula (I) that, when is administrated to a subject, it is able to provide (directly or indirectly) the mentioned compound with formula (I). The term "subject" includes any animal, for example, a mammal, including human beings. The preparation of the mentioned salts can be accomplished by conventional methods known by the technicians in the field. By way of illustration, a salt of a compound with formula (I) can be synthesized by conventional methods from a compound with formula (I) that possess a basic moiety. Generally, those salts are prepared, for example, making react the free base forms of the compounds with formula (I) with a stoichiometric amount of the suitable acid in water or in an organic solvent or in a mixture of water and an organic solvent. In general, non-aqueous media are preferred, such as ether, ethyl acetate, ethanol, isopropanol or acetonitrile.

In a particular realization, the mentioned salts of the compound with formula (I) are pharmaceutically acceptable salts, i.e., salts that can be administrated to a subject and provide a compound of formula (I) in a biological compartment of the mentioned subject. Among the mentioned pharmaceutically acceptable salts acid addition salts are included, for example, the salts formed from organic and inorganic acids, such as bromhydric, chlorhydric, phosphoric, nitric, sulphuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphtalen-disulfonic, oxalic, pivalic, propionic, *p*-toluenesulfonic, succinic, tartaric and similar, as well as the metallic salts, being the metal selected from sodium, potassium, lithium, calcium, magnesium, zinc, aluminium and similar, or the ammonic salts, or a salt formed from organic bases, such as 2-amino-l-butanol, 2-amino-2-ethyl-1,3-propanediol, 2-amino-2-methyl-1,3-propanediol, benzathine, benzyldimethylamine, chloroprocaine, choline, dibenzylmethylamine, diethanolamine, diisopropanolamine, ethylenediamine, dimethyl stearamine, meglumine, 2-methyl-2-amino-1-propanol, a monoamine-glycol, monoethanolamine, monoisopropanolamine, morpholine, N,N-dibenzyl ethylenediamine, N,N-dimethyl-2-amino-2-methyl-1-propanol, N,N-dimethylaniline, procaine, pyridine, quinoline, t-butyl-dimethylamine, triethanolamine, triethylamine, tris hydroxymethyl aminomethane, triisopropanolamine, trimethylamine and similar, and salts with amino acids such as glycine, lysine, arginine, taurine, histamine, alanine, valise, cysteine and similar.

In another particular embodiment, some substituent groups may be added to the compounds object of the invention to render them susceptible to salt formation. For example, acidic functional groups may form stable salts with cations and basic functional groups forming stable salts with acids. Generally, there must be a difference of at least three units in the pKa values of the compound being used as the drug and the counter ion. For derivatives which are very weak bases, the choice to form salts is preferably a strong acid, such as hydrochloric acid (pKa = -6.1), sulphuric acid (pKa1 = -3.0, pKa2 = -1.96), or methanesulfonic acid (pKa = -1.2) to ensure protonation of the compound. Compounds which are more basic may form salts with weak acids, such as phosphoric acid (pKa1 = 2.15, pKa2 = 7.2, pKa3 = 12.38), tartaric acid (pKa = 2.93), acetic acid (pKa = 4.76), and benzoic acid (pKa = 4.2). For compounds which are very weak acids, strongly basic cations such as sodium (pKa = 14.8), potassium (pKa = 16.0) or calcium (pKa = 12.9) are preferred, to ensure deprotonation of the compound. Compounds which are more acidic may form salts with weaker cations, such as zinc (pKa = 8.96), choline (pKa = 18.9) and diethanolamine (pKa = 9.65). Representative functional groups for the formation of stable salts listed as a function of the relative value of their acid / base strength include, but are not limited to, sulfonic acid (pKa1 = -1.2, pKa2 = -0.7), carboxylic acid (pKa = -4.7) and imide (pKa = 8.2).

In another particular embodiment, these salts of the compound of formula (I) are pharmaceutically unacceptable salts, which may be useful in the preparation of pharmaceutically acceptable salts of the compound of formula (I), or prodrugs or solvates thereof.

The term "prodrug" is used, in this disclosure, in the broadest sense, and includes any compound derived from a compound of formula (I) which, when administered to a subject is capable of providing, directly or indirectly, compound of formula (I), or a pharmaceutically acceptable salt thereof, in this subject. Advantageously, this derivative is a compound which increases the bioavailability of the compound of formula (I) when administered to a subject (e.g., by producing that a compound of formula (I) when administered orally to be more readily absorbed by the blood), or that potentiates the release of a compound of formula (I) into a biological compartment (e.g., brain or lymphatic system) relative to the parent compound (without derivatizing). The nature of this derivative is not critical as long as it can be administered to a subject and provides a compound of formula (I) in a biological compartment of this subject. Such derivatives will be apparent to those skilled in the art, and include, depending on the functional groups present in the molecule and without limitation, the following derivatives of the present compounds: esters, amino acid esters, phosphate esters, sulphonate esters of metal salts, carbamates and amides.

The preparation of such prodrugs may be carried out by conventional methods known to those skilled in the art. Such methods will be chosen depending on the derivatization to be introduced into the compound of formula (I). Illustrative examples of some methods for producing prodrugs of active compounds may be found, for example, in Krogsgaard-Larsen et al. "Textbook of Drug Design and Discovery" Taylor & Francis (April 2002). In a particular embodiment, such prodrug is an amide and its preparation is carried out by conventional methods of amide formation, for example, by reacting the compound of formula (I) as the free base with an organic acid or an acid derivative, for example, with a pharmaceutically acceptable organic acid such as acetic, adipic, aspartic, benzene sulfonic, benzoic, citric, ethane sulfonic, formic, fumaric, glutamic, lactic, maleic, malic, malonic, mandelic, methane sulfonic, 1,5- naphthalene disulfonic, oxalic, pivalic, propionic, p-toluene sulfonic, succinic, tartaric and related acids.

The compounds of formula (I), or their salts, may be in crystalline form either as free compounds or as solvates, both forms falling within the scope of the present invention. The term "solvate" as used herein includes any compound formed by combining molecules of a solvent with molecules or ions of a compound of formula (I) or a salt thereof; such solvent may be an organic solvent, for example an alcohol, or an aqueous solvent, for example water, in which case the solvate is called "hydrate".

In a particular embodiment, such solvate is a pharmaceutically acceptable solvate, i.e., which may be administered to a subject and provide (directly or indirectly) a compound of formula (I) or a salt thereof. In another particular embodiment, said solvate is not pharmaceutically acceptable but may be used in the preparation of pharmaceutically acceptable solvates of the compound of formula (I) or salts thereof.

The preparation of such solvates may be carried out by conventional methods known to those skilled in the art, by contacting the compound of formula (I) or a salt thereof with the appropriate solvent.

In a particular embodiment, the compounds of formula (I) or their salts, prodrugs or solvates, will preferably be in a pure or pharmaceutically acceptable form. A pharmaceutically acceptable form excluding standard pharmaceutical additives such as diluents and carriers, and not including materials considered toxic at normal dosage. The level of purity of the compounds will preferably be greater than 50%, more preferably equal to or greater than 70%, even more preferably equal to or greater than 90%. In a preferred embodiment, the purity of the compound of formula (I), or its salts, prodrugs or solvates, will be greater than 95%.

Compounds of the present invention represented by formula (I) described above may include enantiomers depending on the presence of chiral centers or geometric isomers, depending on the presence of multiple bonds (e.g. Z, E). The geometric, enantiomeric or diastereomeric isomers of the compounds of formula (I) and mixtures thereof are within the scope of the present invention.

In another particular embodiment, the compounds subject to this invention provide pharmaceutically acceptable compositions comprising the compounds of formula I with a pharmaceutically acceptable carrier, for example, pharmaceutical composition including one or more compounds of formula I, alone or in combination with one or more additional therapeutic agents as a mixture with pharmaceutically acceptable excipients.

The term "pharmaceutically acceptable excipient" means one or more compatible solids or liquids, diluents or encapsulating substances which are capable of being administered to a subject.

In a preferred particular embodiment, the invention is referred to the compounds of formula (I) wherein:
R is selected from the group consisting of a phenyl aromatic ring optionally substituted by one or two groups independently selected from fluorine, chlorine, (C₁-C₆)alkyl optionally substituted by one, two, or three halogen atoms selected from fluorine, chlorine and bromine; (C₁-C₆)alkoxyl and nitro; or one heterocycle optionally substituted by one or two groups independently selected from fluorine, chlorine, (C₁-C₆)alkyl; alkoxyl and nitro.
R₁ is hydrogen;
R₂ is hydrogen;
R₃ is hydrogen;
R₄ is hydrogen;
R₅ is hydrogen;
R₆ is hydrogen;
R₇ is acyl (C2)
R₈ is hydrogen; and
n is an integer selected from 0, 1 and 2; and
their salts, prodrugs or solvates, preferably, their pharmaceutically acceptable salts.

In another preferred particular embodiment, the invention is referred to the compounds of formula (I) wherein:
R is phenyl optionally substituted by a group selected from fluorine; (C₁-C₆)alkyl optionally substituted by one, two or three halogen atoms selected from fluorine, chlorine and bromine; (C₁-C₆)alkoxyl and nitro;
R₇ is acyl;
R₈ is hydrogen; and
n is 1; and
their salts, prodrugs or solvates, preferably, their pharmaceutically acceptable salts.

In another preferred particular embodiment, the invention is referred to the compounds of formula (I) wherein:
R is phenyl optionally substituted by a group selected from fluorine; (C₁-C₆)alkyl optionally substituted by one, two or three halogen atoms selected from fluorine, chlorine or bromine; (C₁-C₆)alkoxyl and nitro;
R₇ = R₈ is =C=S; and
n is 1; and
their salts, prodrugs or solvates, preferably, their pharmaceutically acceptable salts.

Particularly preferred compounds of formula (I) of the present invention are the following:
- 3-(2-acetamidoethyl)-1*H* indol-5-yl cinnamate
- 3-(2-acetamidoethyl)-1*H*-indol-5-yl (*E*)-3-(3-methoxyphenyl)acrylate
- 3-(2-acetamidoethyl)-1*H*-indol-5-yl (*E*)-3-(4-methoxyphenyl)acrylate
- 3-(2-acetamidoethyl)-1*H*-indol-5-yl (*E*)-3-(2-methoxyphenyl)acrylate
- 3-(2-acetamidoethyl)-1*H*-indol-5-yl (*E*)-3-(4-chlorophenyl)acrylate
- 3-(2-acetamidoethyl)-1*H*-indol-5-yl (*E*)-3-(4-bromophenyl)acrylate
- 3-(2-acetamidoethyl)-1*H*-indol-5-yl (*E*)-3-(4-fluorophenyl)acrylate
- 3-(2-acetamidoethyl)-1*H*-indol-5-yl (*E*)-3-(3-(trifluoromethyl)phenyl)acrylate
- 3-(2-acetamidoethyl)-1*H*-indol-5-yl (*E*)-3-(4-(trifluoromethyl)phenyl)acrylate
- 3-(2-acetamidoethyl)-1*H* indol-5-yl (*E*)-3-(2-(trifluoromethyl)phenyl)acrylate
- 3-(2-acetamidoethyl)-1*H*-indol-5-yl (*E*)-3-(4-methylphenyl)acrylate
- 3-(2-acetamidoethyl)-1*H*-indol-5-yl (*E*)-3-(3,4-dichlorophenyl)acrylate
- 3-(2-acetamidoethyl)-1*H*-indol-5-yl (*E*)-3-(4-nitrophenyl)acrylate
- 3-(2-acetamidoethyl)-1*H*-indol-5-yl (*E*)-2-butenoate
- 3-(2-acetamidoethyl)-1*H*-indol-5-yl (*E*)-3-(4-hydroxyphenyl)acrylate
- 3-(2-acetamidoethyl)-1*H*-indol-5-yl (*E*)-3-(4-hydroxy-3-methoxyphenyl)acrylate

The compounds of formula (I) of the present invention can be obtained following a process, which comprises the reaction of a α,β-unsaturated carboxylic acid of formula (II) 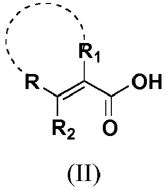 where R, R₁ and R₂ have the meaning previously indicated;
with a alkylamide-5-X indole of formula (III): 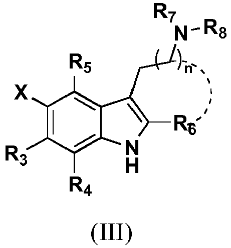 where, R₃, R₄, R₅, R₆, R₇, R₈ and n have the meaning previously indicated.

The reaction is performed in an appropriate inert solvent, at the corresponding temperature in presence of a catalyst. In a particular embodiment, this reaction is performed at room temperature, approximately. In another particular embodiment, this reaction is performed in presence of (1-[bis(dimethylamino)methylene]-1H-1,2,3-triazolo[4,5-b]pyridinium 3-oxid hexafluorophosphate) (HATU), as catalyst and promoter. In another particular embodiment, the reaction is performed in presence of *N,N'*-dicyclohexylcarbodiimide (DCC) as catalyst and promoter. In another particular embodiment, the reaction is performed in presence of 1-ethyl-3-(3-dimethylaminopropyl)carbodiimide (EDCI) as catalyst and promoter. In another particular embodiment, the reaction is performed in an inert solvent, as the formed by an aliphatic halogenated hydrocarbure as: dichloromethane, 1,2-dichloroethane, chloroform, or their mixture, dichloromethane resulting especially adequate. If it is desired, the compound of formula (I) can be converted in a salt, prodrug or solvate by conventional methods as depicted before.

Thus, in another aspect, the present invention describes the procedure for the obtention of a compound of formula (I) that comprises the reaction of the described compound of formula (II) with a compound of formula (III).

The compounds of formula (II) are known and can be obtained from commercial sources or can be prepared by conventional methods.

The compounds of formula (III) are also known and can be obtained from commercial sources or can be prepared by conventional methods (see for example point 1.1).

The compounds of formula (I) obtained by the described protocol, if desired, can be purified by conventional methods as crystallization or chromatography.

The compounds of formula (I) of the present invention exhibit both Nrf2 factor inducing activity and free radical scavenging ability, and potential immunomodulatory effect, these derivatives can therefore be used in the prevention or therapy of neurodegenerative diseases, e.g., AD, PD, ALS and HD, as well as the loss of neurons secondary to autoimmune diseases such as MS and/or in the treatment of other neurodegenerative diseases in which neuronal loss occurs, as in the case of brain ischemia, among others.

For administration to a subject in need of treatment, the compounds of formula (I) of the present invention are conveniently administered formulated with suitable excipients for administration by any suitable route, for example, orally, parenterally, subcutaneously, intramuscularly or rectal, preferably orally route.

Therefore, in other aspects, the invention includes a pharmaceutical composition, hereinafter the pharmaceutical composition of the invention, comprising a compound of formula (I), or a pharmaceutically acceptable salt thereof, together with one or more pharmaceutically acceptable excipients.

In a particular embodiment, the pharmaceutical composition of the invention is presented in a dosage form by oral administration, either in solid or liquid form. Illustrative examples of orally administrable dosage forms include tablets, capsules, granulates, solutions, suspensions, etc., which will include suitable pharmaceutically acceptable excipients, such as binders, diluents, disintegrators, lubricants, humectants, etc., and can be prepared by conventional methods. The pharmaceutical composition of the invention may also be adapted for parenteral administration (e.g., intramuscular, intravenous, etc.), in the form of, for example, sterile solutions, suspensions or lyophilized products in the appropriate dosage form; in such case, such pharmaceutical compositions will include suitable excipients, such as buffers, surfactants, etc. The pharmaceutical composition of the invention may also be adapted for subcutaneous administration in the form of, for example, sterile solutions or suspensions, in the appropriate dosage form; in such case, such pharmaceutical compositions will include suitable excipients, such as buffers, surfactants, etc. Also, the pharmaceutical composition of the invention may be adapted for rectally administration for which it will include suitable excipients compatible with the compounds of formula (I) of the invention. The formulations may be prepared according to conventional methods such as those described in Spanish, European or United States of America pharmacopoeias or similar reference texts, for example "Tratado de Farmacia Galénica", de C. Faulí i Trillo, 10 Edición, 1993, Luzán 5, S.A. de Ediciones.

The compound of formula (I) of the invention will be administered in a therapeutically effective amount which will generally depend on the efficacy of the compound of formula (I) chosen, the severity of the pathology being treated, etc. However, it will typically be administered at daily doses comprised between 0.1 and 100 mg of compound of formula (I) per kg of body weight, more preferably the daily doses will be comprised between 2 and 5 mg/kg of body weight.

In another aspect, the invention protects the use of a pharmaceutically acceptable compound of formula (I), its pharmaceutically acceptable salts, prodrugs or solvates, in the manufacture of a pharmaceutical composition for the prevention or treatment of a neurodegenerative disease, such as a neurodegenerative disease that progress with a deficit of acetylcholine or dopamine, and / or ischemic-cerebral disease (stroke).

Neurodegenerative diseases are those, often of unknown cause, in which the progressive degeneration of the nervous system takes place in some of its parts or in its totality. For a detailed description of them see, for example, the monograph "Enfermedades Neurodegenerativas" Coordinadores José Ma Segovia de Arana y Francisco Mora Teruel, editada por Serie Cientifica Farmaindustria, Madrid, Julio 2002.

Ischemic-cerebrovascular accident is an acute pathology that occurs as a result of the interruption of the blood supply to a part of the brain or when a rupture of a blood vessel with cerebral haemorrhage occurs. Although these diseases are not included in the group of neurodegenerative diseases, it is necessary to take into account that, secondarily to an ischemic-cerebrovascular accident, neurodegeneration also occurs in those affected areas. This is the reason for the utility of the treatment of these diseases with the compounds of the invention.

The administration of the compounds of formula (I) of the invention, their pharmaceutically acceptable salts, prodrugs or solvates, may be carried out alone or in combination with additional drugs, such as drugs useful for the treatment of a neurodegenerative disease or an ischemic-cerebral disease, to provide a combination therapy; such additional drugs may form part of the same pharmaceutical composition of the invention comprising the compound of formula (I) and/or pharmaceutically acceptable salts, prodrugs or solvates thereof, in which case they will be administered simultaneously or sequentially at the administration of the pharmaceutical composition of the invention. Illustrative, but non-limiting examples, of such additional drugs which may be employed to provide a combination therapy include agents such as memantine (a NMDA-approved glutamate receptor blocker for use in the advanced stages of Alzheimer's disease), vitamins, and anti-inflammatory or antidepressant drugs.

### Mode of realization of the invention

The present invention is additionally illustrated by the following examples, which are not intended to be limitative of its protection.

### 1. Obtention of the compounds of the invention

The compounds which biological activity is the object of the present invention were synthetized following organic chemistry procedures:

### 1.1) Preparation of N-(2-(5-hydroxy-1H-indol-3-y1)ethyl)acetamide (compound 17)

Step I: To a suspension of 3-(2-aminoethyl)-1*H*-indol-5-ol hydrochloride (5.00 g, 23.56 mmol) in dry dichloromethane (CH₂Cl₂) (40 mL), under argon, at 0 °C, Et₃N (4.77 g, 47.13 mmol) and acetic anhydride (4.88 g, 47.13 mmol) were added. The resulting mixture was stirred at 0 °C for 4 h, allowed to warm up to room temperature and stirred during 10 h. When completion, the reaction was quenched with NH₄Cl (10 %) and extracted with CH₂Cl₂ (3 X 20 mL). The organic layer was washed with brine, dried over anhydrous MgSO₄, filtrated and concentrated under reduced pressure to afford 3-(2-acetamidoethyl)-1*H*-indol-5-yl acetate as pale yellow oil, used in the next step without further purification.

Step II: To a solution of 3-(2-acetamidoethyl)-1*H*-indol-5-yl acetate in methanol (40 mL), powdered K₂CO₃ (3.26 g, 23.56 mmol) was added. The resulting suspension was stirred at room temperature 1.6 h. When the reaction was completed, water was added and extracted with ethyl acetate. The organic layer was dried over anhydrous MgSO₄, concentrated under reduced pressure and the resultant compound was purified by flash chromatography on silica gel, using CH₂Cl₂/MeOH mixtures (0 - 5 %) as eluent to yield compound **17** as a white solid (3.37 g, 65 % yield). Experimental data were in agreement with previously reported data (Macfarlane y col., 1990, J Chromatogr, 532: 13-25).

### 1.2) General procedure A, for the preparation of the carboxylate esters of family I

To a solution of *N*-(2-(5-hydroxy-1*H*-indol-3-yl)ethyl)acetamide (1 eq) and Et₃N (1.5 eq) in CH₂Cl₂ (0.057 M) under argon, a solution of the corresponding acrylic acid (1.2 eq) and HATU (1.5 eq) in dry CH₂Cl₂ (0.057 M) was added dropwise. The resulting solution was stirred at room temperature. When the reaction was finished (TLC) it was quenched with distilled water and allowed to stir for 15 min. Thereafter, the mixture was extracted three times with CH₂Cl₂ (3 x 20 mL). The organic layer was washed with brine, filtrated and dried over anhydrous MgSO₄. The resulting solid was purified by flash chromatography on silica gel using CH₂Cl₂/MeOH mixtures as eluent.

### Example 1: Preparation of 3-(2-acetamidoethyl)-1H indol-5-yl (E)-2-butenoate (Compound 1).

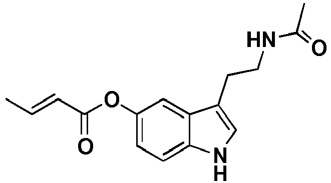

Following the general procedure A, *N*-(2-(5-hydroxy-1*H*-indol-3-yl)ethyl)acetamide (100 mg, 0.458 mmol) and Et₃N (95.8 µL, 0.687 mmol) in CH₂Cl₂ (5 mL) under argon, a solution of (E)-2-butenoic acid (47.3 mg, 0.55 mmol) and HATU (261.2 mg, 0.69 mmol) in CH₂Cl₂ (8 mL), 1.5 h, flash chromatography on silica gel (CH₂Cl₂:MeOH 0:1.5 %) to afford compound **1** as a yellow oil (98 mg, 76 % yield). %). IR (KBr) v 3378, 3278, 2926, 2853, 1730, 1653, 1545, 1314, 1170, 971; ¹H NMR (300 MHz, DMSO-*d₆*) δ_{H} 10.91 (1H, s_{br}, NH), 7.91 (1H, s_{br}, NH), 7.33 (1H, d, *J* = 8.7 Hz, 7-H), 7.24 (1H, d, *J* = 2.2 Hz, 4-H), 7.21 (1H, d, *J* = 2.3 Hz, 2-H), 7.11 (1H, dq, *J* = 15 .6 Hz, *J =* 6.8 Hz, 3'-H), 6. 81 (1 H, dd, *J =* 8.7 Hz, *J =* 2.2 Hz, 6-H), 6.14 (1 H, dd, *J* = 15.6 Hz, *J* = 1.8 Hz, 2'-H), 3.32-3.25 (2H, m, CH₂CH₂NHCOCH₃), 2.78 (2H, t, *J* = 7.4 Hz, CH₂CH₂NHCOCH₃), 1.95 (3H, dd, *J* = 6.8 Hz, *J* = 1.8 Hz, 4'-H), 1.79 (3H, s, CH₂CH₂NHCOCH₃); ¹³C NMR (75 MHz, DMSO-*d₆*) δ_{C} 169.0, 165.0, 146.8, 143.1, 133.9, 127.3, 124.1, 121.9, 115.2, 112.2, 111.6, 110.3, 39.2, 25.0, 22.6, 17.8; HRMS (ES⁺) mass calcd. for C₁₆H₁₈N₂O₃: 286.1317; found [(M+H)⁺] 287.1381; found [(M+Na)⁺] 309.1213; found [(2M+Na)⁺] 595.2549; Anal. calcd. for C₁₆H₁₈N₂O₃ C: 67.12 %; H: 6.34 %; N: 9.78 %; Found: C: 67.30 %; H: 6.29 %; N: 9.54 %.

### Example 2: Preparation of 3-(2-acetamidoethyl)-1H-indol-5-yl cinnamate (Compound 2).

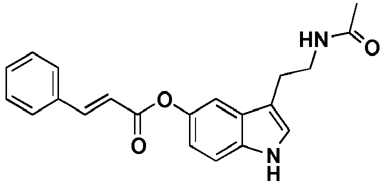

Following the general procedure A, *N*-(2-(5-hydroxy-1*H*-indol-3-yl)ethyl)acetamide (50 mg, 0.23 mmol) and Et₃N (46 µL, 0.34 mmol)) in CH₂Cl₂(5 mL), trans-cinnamic acid (48.9 mg, 0.34 mmol) and HATU (129.3 mg, 0.34 mmol) in CH₂Cl₂ (5 mL), 1.5 h, flash chromatography on silica gel (CH₂Cl₂-MeOH 0-2 %) to afford compound 2 as a white solid (55 mg, 72 % yield); MP: 150-153 °C; IR (KBr) v 3371, 3176, 2922, 1720, 1664, 1565, 1309, 1154, 981, 766; ¹H NMR (300 MHz, CDCl₃) δ_{H} 8.35 (1H, s_{br}, NH), 7.82 (1H, d, *J* = 16.0 Hz, 3'-H), 7.54-7.51 (2H, m, Ph), 7.36-7.34 (3H, m, Ph), 7.27 (1H, d, *J* = 8.6 Hz, 7-H), 7.26 (1H, d, *J* = 2.3 Hz, 4-H), 6.94 (1H, s, 2-H), 6.90 (1H, dd, *J* = 8.6 Hz, *J* = 2.3 Hz, 6-H), 6.60 (1H, d, *J* = 16.0, 2'-H), 6.02 (1H, s_{br}, NH), 3.49-3.43 (2H, m, CH₂*CH*₂NHCOCH₃), 2.84 (2H, t, *J* = 6.7 Hz, CH₂CH₂NHCOCH₃), 1.86 (3H, s, CH₂CH₂NHCO*CH₃*); ¹³C NMR (75 MHz, CDCl₃) δ_{C} 170.7, 166.6, 146.3, 144.2, 134.3, 134.3, 130.6, 129.0, 128.3, 127.7, 123.6, 117.6, 116.2, 113.0, 111.9, 110.8, 40.0, 25.1, 23.0; HRMS (ES⁺) mass calcd. for C₂₁H₂₀N₂O₃ 348.1474; found [(M+H)⁺] 349.1548; found [(M+Na)⁺] 371.1395; Anal. calcd. for C₂₁H₂₀N₂O₃: C: 72.40 %; H: 5.79 %; N: 8.04 %; Found: C: 71.99 %; H: 5.93 %; N: 7.89 %.

### Example 3: Preparation of 3-(2-acetamidoethyl)-1H-indol-5-yl (E)-3-(4-methylphenyl)acrylate (Compound 3).

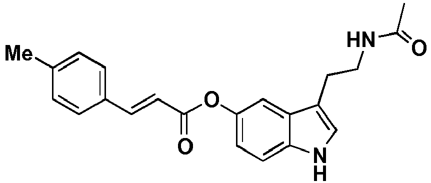

Following general procedure A, *N*-(2-(5-hydroxy-1*H*-indol-3-yl)ethyl)acetamide (100 mg, 0.458 mmol) and Et₃N (95.8 µL, 0.687 mmol)) in CH₂Cl₂ (8 mL), (*E*)*-*3*-*(*p-*tolyl)acrylic acid (0.089 g, 0.550 mmol) and HATU (261.2 g, 0.687 mmol) in CH₂Cl₂ (8 mL), 2.5 h, flash chromatography on silica gel (CH₂Cl₂:MeOH 0-2 %) to afford compound 3 as a brown solid (0.124 g, 75 % yield). MP: 170-172 °C; IR (KBr) v 3344, 3252, 2932, 1703, 1628, 1554, 1483, 1326, 1170, 810. ¹H NMR (CDCl₃, 300 MHz) δ_{H} 8.35 (1H, S_{br}, NH), 7.79 (1H, d, *J* = 16,0, 3'-H), 7.42 (2H, *d, J =* 8.0 Hz, 2"-H), 7.27 (1H, *d, J =* 2.5 Hz, 4-H), 7.25 (1H, d, *J* = 8.9 Hz, 7-H), 7.16 (2H, d, *J* = 8.0 Hz, 3"-H), 6.93 (1H, s, 2-H), 6.90 (1H, dd, *J =* 8.9 Hz, *J =* 2.5 Hz; 6-H), 6.55 (1H, d, *J* = 16.0 Hz, 2'-H), 5.90 (1H, S_{br}, NH), 3.48-3.43 (2H, m, CH₂*CH₂*NHCOCH₃), 2.83 (2H, t, *J* = 7.37 Hz, *CH*₂CH₂NHCOCH₃), 2.33 (3H, s, *CH*₃-Ph), 1.85 (3H, s, CH₂CH₂NHCO*CH₃*). ¹³C NMR (CDCl₃, 75 MHz) δ_{C} 170.7, 166.8, 146.3, 144.2, 141.1, 134.3, 131.5, 129.7, 128.3, 127.7, 123.6, 116.5, 116.2, 112.9, 111.8, 110.8, 39.9, 25.1, 23.0, 21.5. MS (API-ES+) m/z: [(M+H)⁺] 363.1713; [(M+Na)⁺] 385.1517. Anal. calcd. for C₂₂H₂₂N₂O₃: C: 72.91 %; H: 6.12 %; N: 7.73 %; found: C: 72.76 %; H: 6.29 %; N: 7.68 %.

### Example 4: Preparation of 3-(2-acetamidoethyl)-1H-indol-5-yl (E)-3-(2-methoxyphenyl)acrylate (Compound 4).

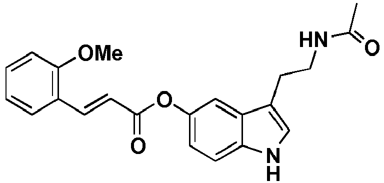

Following general procedure A, *N*-(2-(5-hydroxy-1*H*-indol-3-yl)ethyl)acetamide (50 mg, 0.23 mmol) and Et₃N (48.0 µL, 0.34 mmol) in CH₂Cl₂ (5 mL), (*E*)-3-(2-methoxyphenyl)acrylic acid (48.8 mg, 0.27 mmol) and HATU (129.3 mg, 0.34 mmol) in CH₂Cl₂ (5 mL), 3 h, flash chromatography on silica gel (CH₂Cl₂-MeOH 0-2 %) to afford compound **4** as a white solid (59 mg, 71 % yield); MP: 163-165 °C; IR (KBr) v 3366, 2961, 2924, 1715, 1634, 1487, 1294, 1252, 1158, 765; ¹H NMR (300 MHz, DMSO-*d₆*) δ_{H} 10.93 (1H, S_{br}, NH), 8.07 (1H, d, *J* = 16.2 Hz, 3'-H), 7.91 (1H, S_{br}, NH), 7.81 (1H, dd, *J₆"-₅" =* 7.5 Hz*, J_{6''-4''}*= 1.6 Hz, 6"-H), 7.46 (1H, ddd, *J_{4''-3''}* = 8.5 Hz, *J_{4''-5''}*= 7.5 Hz, *J_{4''-6"}* = 1.6 Hz, 4"-H), 7.36 (1H, d, *J* = 8.6 Hz, 7-H), 7.31 (1H, d, *J* = 2.2 Hz, 4-H), 7.23 (1H, d, *J =* 2.2 Hz, 2-H), 7.13 (1H, d, *J_{3"-4"}* = 8.5 Hz, 3"-H), 7.03 (1H, m, 5"-H), 6.93-6.83 (2H, m, 2'-H, 6-H), 3.90 (3H, s, O*CH₃*), 3.37-3.23 (2H, m, CH₂*CH*₂NHCOCH₃), 2.79 (2H, t, *J* = 7.3 Hz, CH₂CH₂NHCOCH₃), 1.80 (3H, s, CH₂CH₂NHCO*CH₃*); ¹³C NMR (75 MHz, DMSO-*d₆*) δ_{C} 169.0, 166.0, 158.0, 143.2, 140.5, 134.0, 132.3, 128.9, 127.3, 124.1, 122.1, 120.7, 117.7, 115.3, 112.2, 111.8, 111.6, 110.4, 55.7, 39.5, 25.1, 22.6; HRMS (ES⁺) mass calcd. for C₂₂H₂₂N₂O₄ 378.1580; found [(M+H)⁺] 379.1669; found [(M+Na)⁺] 401.1483; found [(2M+Na)⁺] 779.3040; Anal. calcd. for C₂₂H₂₂N₂O₄: C: 69.83 %; H: 5.86 %; N: 7.40 %; found: C: 69.78 %; H: 5.86 %; N: 7.25 %.¹H NMR (300 MHz, DMSO-*d₆*) δ_{H} 10.93 (1H, S_{br}, NH), 8.07 (1H, d, *J* = 16.2 Hz, 3'-H), 7.91 (1H, S_{br}, NH), 7.81 (1H, dd, *J_{6"-5"}* = 7.5 Hz, *J_{6"-4"}* = 1.6 Hz, 6"-H), 7.46 (1H, ddd, *J_{4''-3"}* = 8.5 Hz*, J_{4"-5"}* = 7.5 Hz, *J_{4''-6"}* = 1.6 Hz, 4"-H), 7.36 (1H, d, *J =* 8.6 Hz, 7-H), 7.31 (1H, d, *J =* 2.2 Hz, 4-H), 7.23 (1H, d, *J* = 2.2 Hz, 2-H), 7.13 (1H, d, *J_{3"-4"}* = 8.5 Hz, 3"-H), 7.03 (1H, m, 5"-H), 6.93-6.83 (2H, m, 2'-H, 6-H), 3.90 (3H, s, *OCH₃*)*,* 3.37-3.23 (2H, m, CH₂*CH*₂NHCOCH₃), 2.79 (2H, t, *J =* 7.3 Hz, *CH₂*CH₂NHCOCH₃), 1.80 (3H, s, CH₂CH₂NHCO*CH₃*); ¹³C NMR (75 MHz, DMSO-*d₆*) δ_{C} 169.0, 166.0, 158.0, 143.2, 140.5, 134.0, 132.3, 128.9, 127.3, 124.1, 122.1, 120.7, 117.7, 115.3, 112.2, 111.8, 111.6, 110.4, 55.7, 39.5, 25.1, 22.6; HRMS (ES⁺) mass calcd. for C₂₂H₂₂N₂O₄ 378.1580; found [(M+H)⁺] 379.1669; found [(M+Na)⁺] 401.1483; found [(2M+Na)⁺] 779.3040; Anal. calcd. for C₂₂H₂₂N₂O₄: C: 69.83 %; H: 5.86 %; N: 7.40 %; found: C: 69.78 %; H: 5.86 %; N: 7.25 %.

### Example 5: Preparation of 3-(2-acetamidoethyl)-1H-indol-5-yl (E)-3-(3-methoxyphenyl)acrylate (Compound 5).

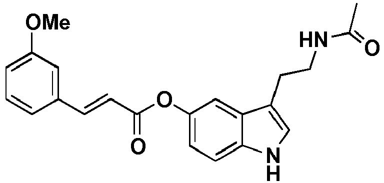

Following general procedure A, *N*-(2-(5-hydroxy-1*H*-indol-3-yl)ethyl)acetamide (50 mg, 0.23 mmol) and Et₃N (47.5 µL, 0.33 mmol) in CH₂Cl₂ (5 mL), (*E*)-3-(3-methoxyphenyl)acrylic acid (48.8 mg, 0.27 mmol) and HATU (129.3 mg, 0.340 mmol) in CH₂Cl₂ (5 mL), 2 h, flash chromatography on silica gel (CH₂Cl₂-MeOH 0-2 %) to afford compound **5** as a white solid (77 mg, 93 % yield); MP: 124-125°C; IR (KBr) v 3381, 3317, 3183, 2928, 1723, 1636, 1488, 1231, 1177, 984, 786. ¹H NMR (300 MHz, CDCl₃) δ_{H} 8.71 (1H, S_{br}, NH), 7.79 (1H, d, *J* = 16.0 Hz, 3'-H), 7.30-7.27 (2H, m, 5"-H, 2"-H), 7.23-7.21 (1H, m, 4"-H), 7.12 (1H, d, *J* = 7.7 Hz, 6"-H) 7.05 (1H, s, 4-H), 6.94-6.86 (3H, m, 6-H, 7-H, 2-H), 6.60 (1H, d, *J* = 16.0 Hz, 2'-H), 6.04 (1H, S_{br}, NH), 3.79 (3H, s, O*CH₃*)*,* 3.45-3.39 (2H, m, CH₂*CH*₂NHCOCH₃), 2.80 (2H, t, *J =* 6.7 Hz, *CH*₂CH₂NHCOCH₃), 1.84 (3H, s, CH₂CH₂NHCO*CH*₃); ¹³C NMR (75 MHz, CDCl₃) δ_{C} 170.8, 166.6, 159.9, 146.3, 144.1, 135.6, 134.4, 130.0, 127.7, 123.7, 121.0, 117.9, 116.6, 116.1, 113.1, 112.9, 111.9, 110.7, 55.4, 40.0, 25.1, 23.0; HRMS (ES⁺) mass calcd. for C₂₂H₂₂N₂O₄ 378.1580; found [(M+H)⁺] 379.1659; found [(M+Na)⁺] 401.1479, found [(2M+Na)⁺] 779.3040; Anal. calcd. for C₂₂H₂₂N₂O₄: C: 69.83 %; H: 5.86 %; N: 7.40 %; found: C: 69.59 %; H: 5.93 %; N: 7.21 %.

### Example 6: Preparation of 3-(2-acetamidoethyl)-1H-indol-5-yl (E)-3-(4-methoxyphenyl)acrylate (Compound 6).

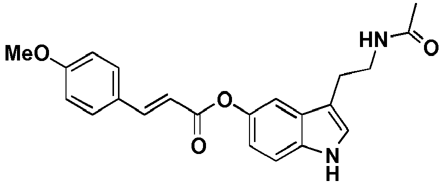

Following general procedure A, *N*-(2-(5-hydroxy-1*H*-indol-3-yl)ethyl)acetamide (50 mg, 0.23 mmol) and Et₃N (47.5 µL, 0.33 mmol) in CH₂Cl₂ (5 mL), (*E*)-3-(4-methoxyphenyl)acrylic acid (48.9 mg, 0.27 mmol) and 0 (129.3 mg, 0.34 mmol) in CH₂Cl₂ (5 mL), 1 h, flash chromatography on silica gel (CH₂Cl₂-MeOH 0-2 %) to afford compound **6** as a white solid (92 mg, 96 % yield); MP: 165-167 °C; IR (KBr) v 3380, 3258, 2935, 1709, 1602, 1427, 1291, 1258, 1152, 868. ¹H NMR (300 MHz, DMSO-*d₆*) δ_{H} 10.94 (1H, S_{br}, NH), 7.93 (1H, S_{br}, NH), 7.83 (1H, d, *J* = 16.1 Hz, 3'-H), 7.77 (2H, d, *J* = 8.8 Hz, 2''-H), 7.37 (1H, d, *J* = 8.8 Hz, 7-H), 7.30 (1H, d, *J* = 2.2 Hz, 4-H), 7.23 (1H, s, 2-H), 7.02 (2H, d, *J* = 8.8 Hz, 3"-H), 6.88 (1H, dd, *J* = 8.8 Hz, *J =* 2.2 Hz, 6-H), 6.75 (1H, d, *J* = 16.1 Hz, 2'-H), 3.83 (3H, s, O*CH₃*)*,* 3.34-3.27 (2H, m, CH₂*CH*₂NHCOCH₃), 2.80 (2H, t, *J* = 7.4 Hz, *CH*₂CH₂NHCOCH₃), 1.80 (3H, s, CH₂CH₂NHCO*CH*₃); ¹³C NMR (75 MHz, DMSO-*d₆*) δ_{C} 169.0, 166.0, 161.3, 145.6, 143.3, 134.0, 130.4, 127.4, 126.6, 124.1, 115.4, 114.9, 114.4, 112.2, 111.6, 110.4, 55.3, 39.5, 25.1, 22.6; HRMS (ES⁺) mass calcd. for C₂₂H₂₂N₂O₄ 378.1580; found [(M+H)⁺] 379.1670; found [(M+Na)⁺] 401.1489; found [(2M+Na)⁺] 779.3049. Anal. calcd. for C₂₂H₂₂N₂O₄: C: 69.83 %; H: 5.86 %; N: 7.40 %; found: C: 69.48 %; H: 5.58 %; N: 7.59%.

### Example 7: Preparation of 3-(2-acetamidoethyl)-1H-indol-5-yl (E)-3-(4-hydroxyphenyl)acrylate (Compound 7).

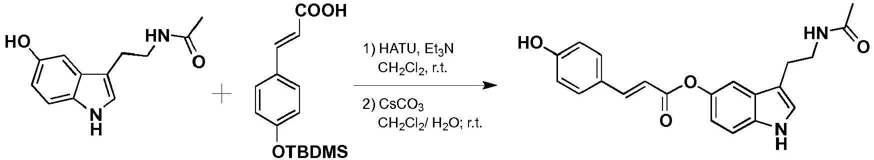

1) Following general procedure A, *N*-(2-(5-hydroxy-1*H-*indol-3-yl)ethyl)acetamide (248.0 mg, 0.90 mmol) and Et₃N (188 µL, 1.35 mmol) in CH₂Cl₂ (10 mL) (*E*)-3-(4-((*tert*-butyldimethylsilyl)oxy)phenyl)acrylic acid (300 mg, 1.08 mmol) and HATU (513.3 mg, 1.35 mmol) in CH₂Cl₂(10 mL), 3 h. After completion of the reaction, resulting crude was subjected to the second step without further purification of the intermediate compound 3-(2-acetamidoethyl)-1*H*-indol-5-yl *(E)*-3-(4-((*tert*-butyldimethylsilyl)oxy)phenyl)acrylate.
2) To a solution of (*E*)-3-(2-acetamidoethyl)-1*H*-indol-5-yl 3-(4-*((tert*butyldimethylsilyl)oxy)phenyl)acrylate (290 mg, 0.6 mmol) in DMF (8 mL), Cs₂CO₃ (99 mg, 0.3 mmol) and H₂O (0.8 mL) was added. The resulting suspension was stirred at room temperature over 15 h. When completion, the reaction mixture was diluted with Et₂O (50 mL) and washed with brine, then extracted with AcOEt (3x10 mL), dried over MgSO₄, concentrated under reduced pressure and purified by flash chromatography on silica gel (CH₂Cl₂-MeOH 0-4 %) to afford the compound 7 as a white solid (290 mg, 61 %). MP: 136-139 °C; IR (KBr) v 3562, 3499, 3364, 2932, 1690, 1597, 1548, 1456, 1327, 1182, 834; ¹H NMR (300 MHz, DMSO-*d₆*) δ_{H} 10.93 (1H, S_{br}, NH), 10.08 (1H, S_{br}, OH), 7.93 (1H, S_{br}, NH), 7.76 (1H, d*, J* = 16.0 Hz, 3'-H), 7.64 (2H, d, *J* = 8.6 Hz, 3"-H), 7.35 (1H, *d, J =* 8.6 Hz, 7-H), 7.28 (1H, d, *J* = 2.2 Hz, 4-H), 7.22 (1H, d, *J* = 2.2 Hz, 2-H), 6.88-6.82 (3H, m, 2"-H, 6-H), 6.65 (1H, d, *J =* 16.0 Hz, 2'-H), 3.32-3.26 (2H, m, CH₂*CH*₂NHCOCH₃), 2.78 (2H, t, *J* = 7.4 Hz, *CH₂*CH₂NHCOCH₃), 1.79 (3H, s, CH₂CH₂NHCO*CH₃*); ¹³C NMR (75 MHz, DMSO-*d₆)* δ_{C} 168.9, 166.1, 160.1, 146.0, 143.3, 133.9, 130.5, 127.3, 125.0, 124.1, 115.8, 115.4, 113.7, 112.2, 111.6, 110.4, 39.4, 25.1, 22.6; HRMS (ES⁺) mass calcd. for C₂₁H₂₀N₂O₄ 364.1423; found [(M+H)⁺] 365.1515; found [(M+Na)⁺] 387.1325; found [(2M+Na)⁺] 751.2731; Anal. calcd. for C₂₁H₂₀N₂O₄: C: 69.22 %; H: 5.53 %; N: 7.69 %; Found: C: 69.33 %; H: 5.75 %; N: 7.94 %.

### Example 8: Preparation of 3-(2-acetamidoethyl)-1H-indol-5-yl (E)-3-(4-hydroxy-3-methoxyphenyl)acrylate (Compound 8).

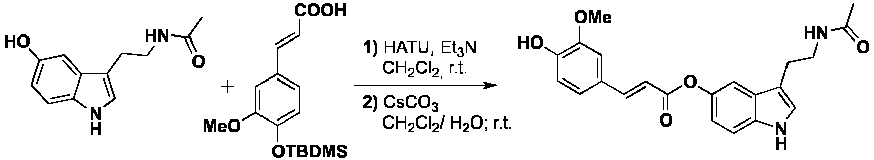

1) Following general procedure A, *N*-(2-(5-hydroxy-1*H-*indol-3-yl)ethyl)acetamide (542 mg, 2.48 mmol) and Et₃N (500 µL, 3.72 mmol) in CH₂Cl₂ (10 mL), (*E*)-3-(4-((*tert*-butyldimethylsilyl)oxy)-3-methoxyphenyl)acrylic acid (920 mg, 2.98 mmol) and HATU (1.414 g, 3.72 mmol) in CH₂Cl₂ (10 mL), 4 h. Once the reaction was finalized, the crude of the reaction was subjected to step 2 without further purification of the intermediate 3-(2-acetamidoethyl)-1*H*-indol-5-yl (*E*)-3-(4-((*tert*-butyldimethylsilyl)oxy)-3-methoxyphenyl)acrylate.
2) To a solution of (*E*)-3-(2-acetamidoethyl)-1*H*-indol-5-yl 3-(4-((*tert*butyldimethylsilyl)oxy)-3-methoxyphenyl)acrylate (600 mg, 1.18 mmol) in CH₂Cl₂(8 mL), C_{S2}CO₃ (192 mg, 0.589 mmol) and H₂O (0.8 mL) was added. The resulting suspension was stirred at room temperature 6 h. When completion, the reaction was diluted with Et₂O (20 mL) and washed with saturated-NaCl. Then extracted with AcOEt (3x10 mL), dried over MgSO₄, concentrated under reduced pressure and purified by flash chromatography on silica gel (CH₂Cl₂:MeOH 0-4 %) to afford compound **8** as a white solid (296 mg, 64 % yield); MP: 193-195 °C; IR (KBr) v 3377, 3265, 3105, 2933, 1704, 1631, 1583, 1514, 1275, 1242, 979, 815; ¹H NMR (300 MHz, DMSO-*d₆*) δ_{H} 10.93 (1H, S_{br}, NH), 9.69 (1H, S_{br}, OH), 7.94 (1H, S_{br}, NH), 7.75 (1H, d, *J* = 16.0 Hz, 3'-H), 7.43 (1H, d, *J* = 1.9, 2"-H), 7.36 (1H, d, *J* = 8.6, 7-H), 7.29 (1H, d, *J* = 2.2 Hz, 4-H), 7.23-7.20 (2H, m, 2-H, 6"-H), 6.89-6.82 (2H, m, 6-H, 5"-H), 6.74 (1H, d, *J* = 16.0 Hz, 2'-H), 3.85 (3H, s, OC*H₃*) 3.31-3.26 (2H, m, CH₂*CH*₂NHCOCH₃), 2.79 (2H, t, *J* = 7.4 Hz, *CH*₂CH₂NHCOCH₃), 1.79 (3H, s, CH₂CH₂NHCO*CH*₃); ¹³C NMR (75 MHz, DMSO-*d₆*) δ_{C} 168.9, 166.1, 149.6, 147,9, 146.3, 143.3, 133.9, 127.3, 125.5, 124.1, 123.4, 115.5, 115.3, 113.9, 112.2, 111.6, 111.4, 110.3, 55.71, 39.4, 25.1, 22.6; HRMS (ES⁺) mass calcd. for C₂₂H₂₂N₂O₅ 394.1529; found [(M+H)⁺] 395.1622; found [(M+Na)⁺] 417.1436; found [(2M+H)⁺] 789.3022; found [(2M+Na)⁺] 811.2916; Anal. calcd. for C₂₂H₂₂N₂O₅ C: 66.99 %; H: 5.62 %; N: 7.10 %; Found: C: 67.05 %; H: 5.96 %; N: 6.66 %.

### Example 9: Preparation of 3-(2-acetamidoethyl)-1H-indol-5-yl (E)-3-(4-fluorophenyl)acrylate (Compound 9).

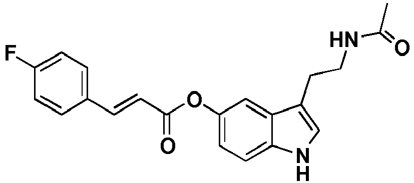

Following general procedure A, *N*-(2-(5-hydroxy-1*H*-indol-3-yl)ethyl)acetamide (100 mg, 0.458 mmol) and Et₃N (95.8 µL, 0.687 mmol) in CH₂Cl₂ dry (8 mL) under argon, a solution of (*E*)-3-(4-fluorophenyl)acrylic acid (91.4 mg, 0.549 mmol) and HATU (261.2 g, 0.687 mmol) in CH₂Cl₂ (8 mL), 2 h, flash chromatography on silica gel (CH₂Cl₂-MeOH 0-2.5 %) to afford compound **9** as a white solid (151 mg, 90 % yield); MP: 173-176 °C; IR (KBr) v 3358, 3217, 2932, 1718, 1636, 1556, 1509, 1314, 1229, 1170, 1103, 982, 830. ¹H NMR (300 MHz, CDCl₃) δ_{H} 8.52 (1H, S_{br}, NH), 7.83 (1H, d, *J* = 16.0 Hz, 3'-H), 7.60-7.55 (2H, m, 2"-H), 7.35-7.32 (2H, m, 7-H, 4-H), 7.14-7.08 (2H, m, 3"-H), 7.00 (1H, s, 2-H), 6.95 (1H, dd, *J =* 8.8 Hz, *J =* 2.2 Hz, 6-H), 6.58 (1H, d, *J* = 16.0 Hz, 2'-H), 6.36 (1H, S_{br}, 1H), 3.53-3.51 (2H, m, CH₂*CH*₂NHCOCH₃), 2.90 (2H, t, *J* = 6.7 Hz, *CH*₂CH₂NHCOCH₃), 1.93 (3H, s, CH₂CH₂NHCO*CH₃*); ¹³C NMR (75 MHz, CDCl₃) δ_{C} 171.1, 166.4, [165.80, 162.46, *J_{C-F}* = 251.4 Hz, C4"], 145.0, 144.2, 134.3, 130.5, [130.27, 130.16, *J_{C-F}* = 8.57 Hz, C2"], 127.7, 123.7, [117.35, 117.32, *J_{C-F}* = 2.23 Hz, C1"], [116.34, 116.04, *J_{C-F} =* 21.7 Hz, C3"], 116.3, 113.0, 111.9, 110.8, 40.2, 25.0, 22.8. HRMS (ES⁺) mass calcd. for C₂₁H₁₉FN₂O₃ 366.1380; found [(M+H)⁺] 367.1479; [(M+Na)⁺] 389.1270; [(2M+Na)⁺] 755.2638; Anal. calcd. for C₂₁H₁₉FN₂O₃: C: 68.84 %; H: 5.23 %; N: 7.65 %; found: C: 68.63 %; H: 5.39 %; N: 7.58 %.

### Example 10: Preparation of 3-(2-acetamidoethyl)-1H-indol-5-yl (E)-3-(4-bromophenyl)acrylate (Compound 10).

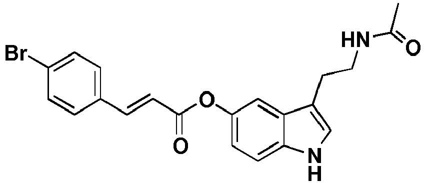

Following general procedure A, N-(2-(5-hydroxy-1H-indol-3-yl)ethyl)acetamide (50 mg, 0.23 mmol) and Et₃N (48.0 µL, 0.34 mmol)) in CH₂Cl₂ (5 mL), (*E*)-3-(4-bromophenyl)acrylic acid (62.4 mg, 0.27 mmol) and HATU (129.3 mg, 0.34 mmol) in CH₂Cl₂ (5 mL), 2.5 h, flash chromatography on silica gel (CH₂Cl₂-MeOH 0-2,5 %) to afford compound **10** as a white solid (96 mg, 97 % yield); MP: 196-198°C; IR (KBr) v 3365, 2928, 2856, 1710, 1637, 1483, 1309, 1070, 827. ¹H NMR (300 MHz, DMSO-*d₆)* δ_{H} 10.95 (1H, s_{br}, NH), 7.93 (1H, s_{br}, NH), 7.84 (1H, d, *J* = 16.0 Hz, 3'-H), 7.78 (2H, d, *J =* 8.5 Hz, 2"-H), 7.67 (2H, d, *J =* 8.5 Hz, 3"-H), 7.37 (1H, d, *J =* 8.6 Hz, 7-H), 7.31 (1H, d, *J* = 2.3 Hz, 4-H), 7.23 (1H, d, *J* = 2.3 Hz, 2-H), 6.95 (1H, d, *J* = 16.0 Hz, 2'-H), 6.89 (1H, dd, *J* = 8.6 Hz, *J* = 2.3 Hz, 6-H), 3.31-3.26 (2H, m, CH₂*CH*₂NHCOCH₃), 2.79 (2H, t, *J* = 7.4 Hz, *CH*₂CH₂NHCOCH₃), 1.79 (3H, s, CH₂CH₂NHCO*CH*₃); ¹³C NMR (75 MHz, DMSO-*d₆*) δ_{C} 168.9, 165.5, 144.4, 143.1, 134.0, 133.3, 131.9, 130.4, 127.3, 124.2, 124.1, 118.6, 115.2, 112.2, 111.7, 110.3, 39.4, 25.1, 22.6. HRMS (ES⁺) mass calcd. for C₂₁H₁₉ClN₂O₃ 426.0579; found [(M+H)⁺] 427.0655; [(M+Na)⁺] 449.0478; Anal. calcd. for C₂₁H₁₉BᵣN₂O₃: C: 59.03 %; H: 4.48 %; N: 6.56 %; found: C: 58.78 %; H: 4.65 %; N: 6.39 %.

### Example 11: Preparation of 3-(2-acetamidoethyl)-1H-indol-5-yl (E)-3-(4-chlorophenyl)acrylate (Compound 11).

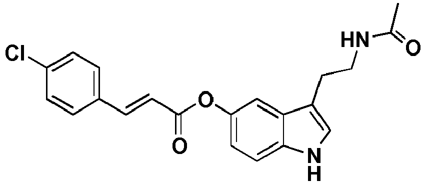

Following general procedure A, *N*-(2-(5-hydroxy-1*H*-indol-3-yl)ethyl)acetamide (50 mg, 0.23 mmol) and Et₃N (48.0 µL, 0.34 mmol) in CH₂Cl₂ (5 mL), (*E*)-3-(4-chlorophenyl)acrylic acid (50.2 mg, 0.27 mmol) and HATU (129.3 mg, 0.34 mmol) in CH₂Cl₂, 3 h, flash chromatography on silica gel (CH₂Cl₂-MeOH 0-2.5 %) to afford compound **11** as a white solid (83 mg, 98 % yield); MP: 185-187 °C; IR (KBr) v 3363, 2928, 1710, 1655, 1550, 1325, 1173, 828; ¹H NMR (300 MHz, DMSO-*d₆*) δ_{H} 10.95 (1H, s_{br}, NH), 7.93 (1H, s_{br}, NH), 7.88-7.83 (3H, m, 3'-H, 2"-H), 7.52 (2H, d, *J* = 8.5 Hz, 3"-H), 7.36 (1H, d, *J* = 8.7 Hz, 7-H), 7.30 (1H, d, *J* = 2.2, 4-H), 7.23 (1H, d, *J =* 2.3 Hz, 2-H), 6.96-6.86 (2H, m, 2'-H, 6-H), 3.33-3.24 (2H, m, CH₂*CH*₂NHCOCH₃), 2.78 (2H, t, *J =* 7.4 Hz, *CH*₂CH₂NHCOCH₃), 1.79 (3H, s, CH₂CH₂NHCO*CH₃*); ¹³C NMR (75 MHz, DMSO-*d₆*) δ_{C} 169.0, 165.6, 144.3, 143.2, 135.2, 134.0, 132.9, 130.3, 129.0, 127.3, 124.2, 118.5, 115.2, 112.2, 111.7, 110.3, 39.7, 25.1, 22.6; HRMS (ES⁺) mass calcd. for C₂₁H₁₉ClN₂O₃ 382.1084; found [(M+H)⁺] 383.1174; found [(M+Na)⁺] 405.0989; Anal. calcd. for C₂₁H₁₉ClN₂O₃: C: 65.88 %; H: 5.00 %; N: 7.32 %; found: C: 65.78 %; H: 5.33 %; N: 6.99 %.

### Example 12: Preparation of 3-(2-acetamidoethyl)-1H-indol-5-yl (E)-3-(3,4-dichlorophenyl)acrylate (Compound 12).

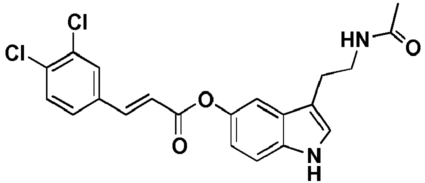

Following general procedure A, *N*-(2-(5-hydroxy-1*H*-indol-3-yl)ethyl)acetamide (100 mg, 0.458 mmol) and Et₃N (95.8 µL, 0.687 mmol) in CH₂Cl₂ (8 mL), (*E*)-3-(3,4-dichlorophenyl)acrylic acid (119.4 mg, 0.550 mmol) and HATU (261.2 mg, 0.687 mmol) in CH₂Cl₂, 1.5 h, flash chromatography on silica gel (CH₂Cl₂-MeOH 0-2 %) to afford compound **12** as a white solid (169 mg, 88 % yield). MP: 167-169 °C; IR (KBr) v 3298, 2943, 1721, 1630, 1540, 1471, 1260, 1238, 1201, 979, 816; ¹H NMR (300 MHz, DMSO-*d₆*) δ_{H} 10.95 (1H, s_{br}, NH), 8.17 (1H, d, *J =* 1.9, 2"-H), 7.93 (1H, s_{br}, NH), 7.89-7.81 (2H, m, 6"-H, 3'-H), 7.72 (1H, *d, J =* 8.3 Hz, 5"-H), 7.37 (1H, d, *J =* 8.6 Hz, 7-H), 7.32 (1H, d, *J =* 2.2 Hz, 4-H), 7.24 (1H, d, *J =* 2.2, 2-H), 7.04 (1H, d, *J* = 16.0, 2'-H), 6.89 (1H. dd, *J* = 8.6 Hz, *J* = 2.2 Hz, 6-H), 3.35-3.26 (2H, m, CH₂*CH*₂NHCOCH₃), 2.80 (2H, t, *J* = 7.3 Hz, *CH*₂CH₂NHCOCH₃), 1.80 (3H, s, CH₂CH₂NHCO*CH*₃); ¹³C NMR (75 MHz, DMSO-*d₆*) δ_{C} 168.9, 165.4, 143.1, 143.0, 134.8, 134.0, 132.9, 131.8, 131.0, 130.3, 128.4, 127.3, 124.2, 120.0, 115.2, 112.3, 111.7, 110.3, 39.5, 25.1, 22.6; HRMS (ES⁺) mass calc'd. For C₂₁H₁₈Cl₂N₂O₃ 416.0694; found [(M+H)⁺] 417.0722; found [(M+Na)⁺] 439.0612; found [(2M+Na)⁺] 855.1218; Anal. calcd. for C₂₁H₁₈Cl₂N₂O₃: C: 60.45 %; H: 4.35 %; N: 6.71 %; found: C: 60.07 %; H: 4.56 %; N: 6.46 %.

### Example 13: Preparation of 3-(2-acetamidoethyl)-1H-indol-5-yl (E)-3-(2-(trifluoromethyl)phenyl)acrylate (Compound 13).

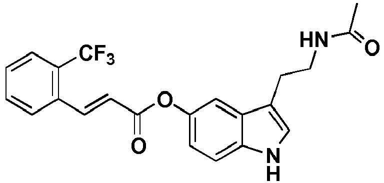

Following general procedure A, *N*-(2-(5-hydroxy-1*H*-indol-3-yl)ethyl)acetamide (100 mg, 0.458 mmol) and Et₃N (95.8 µL, 0.687 mmol)) in CH₂Cl₂ (8 mL), (*E*)-3-(2-(trifluoromethyl)phenyl)acrylic acid (118.8 g, 0.550 mmol) and HATU (261.2 mg, 0.687mmol) in CH₂Cl₂ (8 mL), 3.5 h, flash chromatography on silica gel (CH₂Cl₂-MeOH 0-2 %) to afford compound **13** as a white solid (184 mg, 97 % yield). MP: 145-147 °C; IR (KBr) v 3373, 3174, 1717, 1657, 1577, 1485, 1315, 1244, 1165, 1061, 960, 768. ¹H NMR (300 MHz, CDCl₃) δ_{H} 8.34 (1H, s_{br}, NH), 8.27 (1H, dd, *J* = 16.0 Hz; *J* = 2.2 Hz, 3'-H), 7.81-7.73 (2H, m, 4"-H, 5"-H), 7.65-7.59 (1H, m, 3"-H), 7.54-7.49 (1H, m, 6"-H), 7.40-7.29 (2H, m, 4-H, 7-H), 7.07-6.92 (2H, s, 2-H, 6-H), 6.64 (1H, d, *J* = 16.0 Hz, 2'-H), 5.59 (1H, S_{br}, NH), 3.57-3.51 (2H, m, CH₂*CH*₂NHCOCH₃), 2.91 (2H, t, *J* = 6.7 Hz, *CH*₂CH₂NHCOfCH₃), 1.92 (3H, s, CH₂CH₂NHCO*CH*₃); ¹³C NMR (75 MHz, CDCl₃) δ_{C} 170.8, 165.7, 144.0, 141.7, 134.3, 133.1, 132.2, 129.9, [129.59, 129.19, 128.78, 128.38, *J*_{*C*-*F*} = 30.4, C2"], 128.0, 127.7, [126.37, 126.30, 126.23, 126.15, *J_{C-F}* = 5.7, C3"], [129.34, 125.76, 122.13, 118.50, *J_{C-F}* = 273.9, *CF₃*], 123.7, 121.9, 116.0, 113.0, 111.9, 110.7, 40.0, 25.1, 23.0; HRMS (ES⁺) mass calcd. for C₂₂H₁₉F₃N₂O₃ 416.1348; found [(M+H)⁺] 417.1411; found [(M+Na)⁺] 439.1228; Anal. calcd. for C₂₂H₁₉F₃N₂O₃: C: 63.46 %; H: 4.60 %; N: 6.73 %; found: C: 63.16 %; H: 4.64 %; N: 6.37 %.

### Example 14: Preparation of 3-(2-acetamidoethyl)-1H-indol-5-yl (E)-3-(3-(trifluoromethyl)phenyl)acrylate (Compound 14).

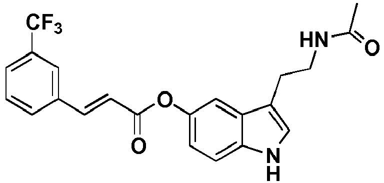

Following general procedure A, *N*-(2-(5-hydroxy-1*H*-indol-3-yl)ethyl)acetamide (100 mg, 0.458 mmol) and Et₃N (95.8 µL, 0.687 mmol) in CH₂C1₂ (8 mL), (*E*)-3-(3-(trifluoromethyl)phenyl)acrylic acid (118.9 mg, 0.550 mmol) and HATU (261.2 mg, 0.687 mmol) in CH₂C1₂, 3 h, flash chromatography on silica gel (CH₂Cl₂:MeOH 0-2 %) to afford compound **14** as a white solid (0.143 g, 75 % yield); MP: 137-139 °C; IR (KBr) v 3374, 3154, 1658, 1641, 1338, 1197, 809, 695. ¹H NMR (300 MHz, CDCl₃) δ_{H} 8.57 (1H, s_{br}, NH), 7.80 (1H, d, *J* = 16.0 Hz; 3'-H), 7.75 (1H, s, 2"-H), 7.67 (1H, d, *J* = 7.7 Hz, 4"-H), 7 .5 9 (1H, d, *J* = 7.7 Hz, 6"-H), 7.47 (1H, t, *J* = 7.7 Hz, 5"-H), 7.26-7.18 (2H, m, 4-H, 7-H), 6.89-6.86 (2H, m, 2-H, 6-H), 6.65 (1H, d, *J* = 16.0 Hz, 2'-H), 5.98 (1H, S_{br}, NH), 3.46-3.40 (2H, m, CH₂CH₂NHCOCH₃), 2.80 (2H, t, *J* = 6.8 Hz, CH₂CH₂NHCOCH₃), 1.83 (3H, s, CH₂CH₂NHCOCH₃); ¹³C NMR (75 MHz, CDCl₃), δ_{C} 170.7, 166.0, 144.4, 144.0, 135.1, 134.4, [132.20, 131.77, 131.34, 130.91, *J_{C-F}* = 32.6, C3"], 131.2, 129.6, [129.18, 125.57, 121.96, 118.35, *J_{C-F}* = 272.4, *CF₃*], 127.7, [127.03, 126.98, 126.93, 126.89, *J_{C-F}* = 3.8, C2"], [124.90, 124.85, 124.80, 124.75, *J_{C-F}* = 3.8, C4"], 123.7, 119.6, 116.0, 113.0, 111.9, 110.7, 40.0, 25.1, 23.0; HRMS (ES⁺) mass calcd. for C₂₂H₁₉F₃N₂O₃ 416.1348; found [(M+H)⁺] 417.1411; found [(M+Na)⁺] 439.1234; Anal. calcd. for C₂₂H₁₉F₃N₂O₃: C: 63.46 %; H: 4.60 %; N: 6.73 %; found: C: 63.36 %; H: 4.77 %; N: 6.41 %.

### Example 15: Preparation of 3-(2-acetamidoethyl)-1H-indol-5-yl (E)-3-(4-(trifluoromethyl)phenyl)acrylate (Compound 15).

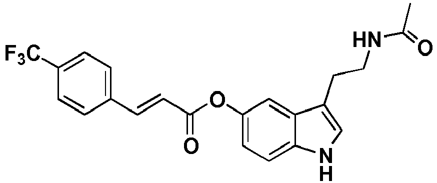

Following general procedure A, *N*-(2-(5-hydroxy-1*H*-indol-3-yl)ethyl)acetamide (100 mg, 0.458 mmol) and Et₃N (95.8 µL, 0.687 mmol) in CH₂C1₂ (8 mL), (*E*)-3-(4-(trifluoromethyl)phenyl)acrylic acid (118.8 mg, 0.550 mmol) and HATU (261.2 mg, 0.687 mmol) in CH₂C1₂ (8 mL), 2 h, flash chromatography on silica gel (CH₂Cl₂-MeOH 0-2 %) to afford compound **15** as a white solid (141 mg, 74 % yield); MP: 184-186 °C; IR (KBr) ν 3356, 3080, 1718, 1655, 1553, 1481, 1325, 1177, 1066, 838. ¹H NMR (300 MHz, CDCl₃) δ_{H} 8.37 (1H, s_{br}, NH), 7.89 (1H, d, *J* = 16.0 Hz, 3'-H), 7.69 (4H, s, 2"-H, 3"-H), 7.39-7.34 (2H, m, 4-H, 7-H), 7.05 (1H, s, 2-H), 6.98 (1H, dd, *J* = 8.7 Hz, *J* = 2.1 Hz, 6-H), 6.74 (1H, d, *J* = 16.0 Hz, 2'-H), 6.14 (1H, s_{br}, NH), 3.59-3.46 (2H, m, CH₂CH₂NHCOCH₃), 2.93 (2H, t, *J* = 6.6 Hz, CH₂CH₂NHCOCH₃), 1.95 (3H, s, CH₂CH₂NHCOCH₃); ¹³C NMR (75 MHz, CDCl₃) δ_{C} 165.9, 144.3, 144.1, 137.6, 135.3, 134.3, [132.76, 132.35, 131.86, 131.39, *J_{C-F}* = 32.6 Hz, C4"], [129.22, 125.61, 122.00, 118.35, *J_{C-F}* = 272.5 Hz, *CF₃*], 128.4, 127.7, [126.06, 126.00, 125.96, 125.90, *J_{C-F}* = 3.82 Hz, C3"], 123,64, 120.24, 116.2, 113.2, 111.9, 110.8, 40.1, 25.0, 22.9; HRMS (ES⁺) mass calcd. for C₂₂H₁₉F₃N₂O₃ 416.1348; found [(M+H)⁺] 417.1418; found [(M+Na)⁺] 439.1238; Anal. calcd. for C₂₂H₁₉F₃N₂O₃: C: 63.46 %; H: 4.60 %; N: 6.73 %; found: C: 63.11 %; H: 4.66 %; N: 6.44 %.

### Example 16: Preparation of 3-(2-acetamidoethyl)-1H-indol-5-yl (E)-3-(4-nitrophenyl)acrylate (Compound 16).

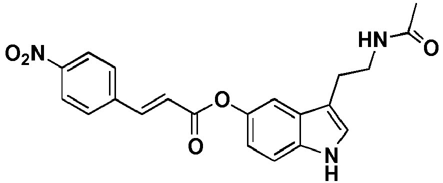

Following general procedure A, *N*-(2-(5-hydroxy-1*H*-indol-3-yl)ethyl)acetamide (100 mg, 0.458 mmol) and Et₃N (95.8 µL, 0.687 mmol)) in CH₂Cl₂ (8 mL) under argon, a solution of (*E*)-3-(4-nitrophenyl)acrylic acid (105.9 mg, 0.550 mmol) and HATU (261.2 g, 0.687 mmol) in CH₂C1₂ (8 mL), 1 h, flash chromatography on silica gel (CH₂Cl₂-MeOH 0-2 %) to afford compound **16** as a yellow solid (146 mg, 81 % yield). MP: 218-220 °C; IR (KBr) v 3380, 3226, 3068, 2924, 1713, 1639, 1343, 1177, 848; ¹H NMR (300 MHz, DMSO-*d₆*) δ_{H} 10.97 (1H, s_{br}, NH), 8.28 (2H, d, *J* = 8.8 Hz, 2"-H), 8.10 (2H, d, *J* = 8.8 Hz, 3"-H), 7.98 (1H, d, *J* = 16.0 Hz, 3'-H), 7.92 (1H, s_{br}, NH), 7.38 (1H, d, *J* = 8.6 Hz, 7-H), 7.34 (1H, d, *J* = 2.2 Hz, 4-H), 7.24 (1H, d, *J* = 2.2 Hz, 2-H), 7.13 (1H, d, *J* = 16.0 Hz, 2'-H), 6.91 (1H, dd, *J* = 8.6 Hz, *J* = 2.2 Hz, 6-H), 3.34-3.25 (2H, m, CH₂CH₂NHCOCH₃), 2.80 (2H, t, *J* = 7.3 Hz, CH₂CH₂NHCOCH₃), 1.80 (3H, s, CH₂CH₂NHCO*CH₃*); ¹³C NMR (75 MHz, DMSO-*d₆*) δ_{C} 169.0, 165.2, 148.2, 143.1, 143.1, 140.4, 134.1, 129.6, 127.3, 124.2, 123.9, 122.0, 115.1, 112.3, 111.7, 110.3, 39.5, 25.1, 22.6; HRMS (ES⁺) mass calc'd. for C₂₁H₁₈N₃O₅ 393.1325; found [(M+H)⁺] 394.1416; found [(M+Na)⁺] 416.1215; found [(2M+Na)⁺] 809.2430; Anal. calcd. for C₂₁H₁₈N₃O₅: C: 64.12 %; H: 4.87 %; N: 10.68 %; found: C: 64.44 %; H: 5.04%; N: 10.59%.

### 2. Biological activities studied with the compounds of the invention

### Example 17

### Nrf2-ARE induction measurement using Luciferase activity in MCF7 cells stable transfected with the plasmid of ARE-LUC

In the human being, the transcriptional factor Nrf2 is codified by NFEL2 gen. In basal conditions, Nrf2 is sequestered in the cytoplasm by the repressor protein Keap1. Keap1 works as substrate protein for the ubiquitinization joining to the protein cullin-3 ubiquitin-ligase (CuI3), thus degrading Nrf2 *via* proteasome. Oxidative stress or electrophilic compounds, modify certain cysteine groups in Keap1 and, as a result, they block the ubiquitinization of the complex Keap1-Cul3, releasing Nrf2 to the cytoplasm. Once released, Nrf2 translocates to the nucleus where it heterodimerizes with Maf proteins and binds to the antioxidant response element (ARE) located in the promoter region of diverse cytoprotective proteins, thus inducing the overexpression of those proteins. These enzymes include the NAD(P)H quinone oxidoreductase, the glutamate-cysteine ligase, the heme-oxygenase 1 and the family of glutathione S-transferase (GST), among others. Nrf2 inductors increase either Nrf2 levels, its activity, or its translocation to the nucleus; consequently, Nrf2 inductors increase the expression of genes regulated via Nrf2.

For the study of Nrf2 transcription factor, the cellular line AREC32 was used, which is characterized for being stably transfected with the reporter gene of Luciferase linked to the ARE sequence. In the presence electrophilic compounds or oxidative stress, Nrf2 factor binds to ARE sequences, activating luciferase expression and producing a proportional quantity of luciferase. The AREc32 cells were cultured in DMEM media with glutamax, supplemented with 10 % fetal bovine serum (FBS), 1 % penicillin-streptomycin and 1.6 % geneticine (G418) (obtained from GIBCO, Madrid, Spain). Cells were cultured in 75 cm flasks with 11 mL of specific media, incubated at 37 °C and 5 % of CO₂. Cells were passed every 4-6 days (1:4), whenever the confluence reached 80 %. For the experiments, cells were cultured in transparent surface 96 well plates at a density of 60x10⁴ cells/well with 100 µL/well. After 24 h in culture, cells were treated with the compounds at different concentrations (1, 10, 30 and 60 µM) during 24 h at 37 °C and 5 % CO₂. In every experiment it was also included a basal variable, a variable with 10 µM tert-butyl hydroquinone (TBHQ) (positive control) and the variables of the study in a final volume of 100 µL. After an incubation of 24 h with the compounds, luciferase activity was measured by bioluminescence using the commercial kit "Luciferase assay system" (Promega E1500). For that, treatments were removed and cells were washed with 100 µL of PBS 0,1 M. After washing, 20 µL of "lysis buffer" reactive was added to each well. 10 minutes later, luminescence was measured using the multi-well reader FluoStar optima (BMG labtech).

**Table 1**

| **Nrf2 transcription factor induction by the invention compounds and TBHQ, compound of reference. Data are presented as media ± S.E. of at least three independent experiments performed in duplicates at four different concentrations.** | | | | | |
|---|---|---|---|---|---|
| **Compound** | **R** | **Relative Luciferase activity (response versus basal)** | | | |
| **Concentration** | | ***1 µM*** | ***3 µM*** | ***6 µM*** | ***8 µM*** |
| **TBHQ** | | | 2.19 ± 0.1** | 3.83 ± 0.3*** | 6.10 ± 0.1*** |
| **Concentration** | | | *10 µM* | *30 µM* | *100 µM* |
| **Melatonin** | | | 1.40 ± 0.1 | 1.64 ± 0.1 | 2.22 ± 0.1 |
| **Concentration** | | | *10 µM* | *30 µM* | *60 µM* |
| **Ethyl Cinnamate** | | | 1.46 ± 0.1*** | 1.40 ± 0.1*** | 1.55 ± 0.1*** |
| **1** | Me | 2.34 ± 0.2** | 2.34 ± 0.2** | 12.1 ± 1.3*** | 30.7 ± 1.7*** |
| **2** | Ph | 1.63 ± 0.2* | 1.63 ± 0.2* | 1.78 ± 0.1** | 2.54 ± 0.3*** |
| **3** | *p*-Me-Ph | 1.26 ± 0.1 | 1.26 ± 0.1 | 2.03 ± 0.1*** | 3.26 ± 0.3*** |
| **4** | *o*-MeO-Ph | 1.67 ± 0.1** | 1.67 ± 0.1** | 2.01 ± 0.1*** | 2.53 ± 0.1*** |
| **5** | *m*-OMe-Ph | 1.39 ± 0.1* | 1.39 ± 0.1* | 1.63 ± 0.2** | 2.09 ± 0.2*** |
| **6** | *p*-OMe-Ph | 2.02 ± 0.2** | 2.02 ± 0.2** | 3.40 ± 0.3*** | 4.62 ± 0.4*** |
| **7** | *p*-OH-Ph | 0.95 ± 0.1 | 0.95 ± 0.1 | 1.09 ± 0.1 | 1.57 ± 0.1*** |
| **8** | *m*-MeO, p-OH-Ph | 1.11 ± 0.1 | 1.11 ± 0.1 | 1.83 ± 0.1*** | 2.36 ± 0.1*** |
| **9** | *p*-F-Ph | 1.92 ± 0.2* | 1.92 ± 0.2* | 2.27 ± 0.2** | 4.21 ± 0.6*** |
| **10** | *p*-Br-Ph | 1.53 ± 0.2** | 1.53 ± 0.2** | 3.12 ± 0.5*** | 13.3 ± 2.1*** |
| **11** | *p*-Cl-Ph | 1.18 ± 0.1 | 1.18 ± 0.1 | 1.59 ± 0.2** | 3.55 ± 0.9*** |
| **12** | *m,p*-di-Cl -Ph | 1.16 ± 0.1 | 1.16 ± 0.1 | 1.43 ± 0.1* | 2.12 ± 0.2*** |
| **13** | *o*-CF₃-Ph | 1.38 ± 0.1* | 1.38 ± 0.1* | 1.79 ± 0.1*** | 2.55 ± 0.2*** |
| **14** | *m*-CF₃-Ph | 1.34 ± 0.2* | 1.34 ± 0.2* | 1.64 ± 0.2** | 2.13 ± 0.2*** |
| **15** | *p*-CF₃-Ph | 2.34 ± 0.2* | 2.34 ± 0.2* | 4.99 ± 0.5*** | 28.9 ± 2.9*** |
| **16** | *p*-NO₂-Ph | 1.69 ± 0.1*** | 1.69 ± 0.1*** | 2.41 ± 0.1*** | 3.19 ± 0.1*** |

Luminescence was measured in arbitrary units and is directly proportional to luciferase quantity in the wells, which is also directly proportional to Nrf2 transcription factor induction by the specific compound at the established concentration. Measurements were made in duplicate and values were normalized to the basal luminescence value, considered as 1.

The results of Nrf2 induction obtained for the compounds 1 to 16 are shown in Table 1 and are expressed as induction, considering 1 the non-treated cells.

### Example 18

### Measurement of the antioxidant capacity: hydroxyl radical scavenger capacity by the compounds of the invention

In order to study the potential antioxidant effect of the compounds of the invention, we performed an oxygen radical absorbance capacity (ORAC) experiment. The compounds of the present invention were designed as hybrids of melatonin, which is a natural compound characterized by its potent free radical scavenger capacity as well as an indirect antioxidant effect via modulation of different signalling pathways (Tan y col., 2002, Curr Top Med Chem, 2: 181-97). Both melatonin and its different intermediate metabolites, as *N-*acetyl-*N-*formil-5- methoxykynuramine or 3-hydroxymelatonin, are able to detoxify free radicals through the antioxidant cascade of melatonin (Burkhardt y col., 2001, Int J Biochem Cell Biol, 33: 775-83, Seegar y col., 1997, Br J Clin Pharmacol, 44: 283-4, Tan y col., 2000, Free Radic Biol Med, 29: 1177-85). The fact that not only melatonin but also its intermediate metabolites present an antioxidant capacity, confers this molecule a better efficiency compared to other antioxidants. Given this mechanism of action, melatonin is highly expressed in tissues and organs frequently exposed to environmental stress, such as the skin, and in organs characterized by a high oxygen consumption, such as the brain.

For studying the free radical scavenger capacity, we measured the degree peroxide radical inhibition. Thus, scavenger capacity is measured as trolox equivalents and includes the disappearance of free radicals over time and the decrease of oxidative damage at the end of the experiment. The protocol used is based in the protocol developed by Ou and col. (Ou y col., 2001, J Agric Food Chem, 49: 4619-26) partially modified by Dávalos and col. (Davalos y col., 2004, J Agric Food Chem, 52: 48-54). The reaction was performed in phosphate tampon 75 mM (pH 7.4), with a final volume of 200 µL. 150 µL of fluorescein (final concentration of 70 nM) with 25 µL the compound of study at the desired concentration in triplicates, using surface transparent 96 well black plates for measuring fluorescence.

**Table 2**

| **Measurements of the free radical scavenger capacity by the compounds of the invention, and melatonin, as reference compound. Data are presented as media ± S.E.M of at least three independent experiments performed in duplicates.** | | |
|---|---|---|
| **Compound** | **R** | **ORAC (Trolox eq.)** |
| **Melatonin** | | **2.18 ± 0.14** |
| **1** | Me | 8.64 ± 1.98 |
| **2** | Ph | 4.30 ± 0.50 |
| **3** | *p*-Me-Ph | 4.44 ± 0.51 |
| **4** | *o*-MeO-Ph | 3.02 ± 0.20 |
| **5** | *m*-OMe-Ph | 3.52 ± 0.27 |
| **6** | *p*-OMe-Ph | 3.51 ± 0.27 |
| **7** | *p*-OH-Ph | 9.21 ± 0.81 |
| **8** | *m*-MeO, *p*-OH-Ph | 8.04 ± 0.66 |
| **9** | *p*-F-Ph | 3.44 ± 0.67 |
| **10** | *p*-Br-Ph | 2.53 ± 0.18 |
| **11** | *p*-Cl-Ph | 2.91 ± 0.32 |
| **12** | *m,p*-di-Cl-Ph | 5.37 ± 0.38 |
| **13** | *o*-CF₃-Ph | 3.33 ± 0.28 |
| **14** | *m*-CF₃-Ph | 4.13 ± 0.39 |
| **15** | *p*-CF₃-Ph | 3.97 ± 0.38 |
| **16** | *p*-NO₂-Ph | 4.12 ± 0.49 |

The mixture was pre-incubated during 15 min at 37 °C and, afterwards, 25 µL of a solution containing 2,2'-Azobis(2-amidinopropane) dihydrochloride (AAPH) (final concentration; 12 mM) were rapidly added. Fluorescence was immediately measured using a plate reader fluostar optima, using the filters of emission and excitation of 485 y 520 nM, respectively. Fluorescence intensity was measured during 90 min. Each compound was measured at 6 different concentrations. In the assay, fluorescein + AAPH in phosphate tampon was added as a blank and a calibration curve was performed using Trolox (1-8 µM). Every variable was prepared in duplicates and at least three independent experiments were performed for each compound. The curves of the compounds (F.I. - time) were normalized respect to the curve of the blank for each experiment and area under the curve (AUC) of the decreasing in fluorescence was calculated. AUC value of each compound was calculated subtracting the AUC of the blank. Regression curves between AUC and concentration of the compound were calculated for each of the samples. ORAC values were expressed as trolox equivalents with respect to the calibration curve of each assay, where the trolox value was considered 1.

### Example 19

### Study of the neuroprotective capacity of the compounds of the invention using different models of toxicity induced by oxidative stress

### SH-SY5Y neuroblastoma cell line culture

SH-SY5Y cells [ECACC 94030304], in 5 to 16 passage after defreezing were maintained in Eagle media modified by Dulbecco (DMEM) containing 15 non-essential amino acids and supplemented with 10 % fetal bovine serum, 1 mM glutamine, 50 units/mL penicillin/streptomycin (obtained from GIBCO, Madrid, Spain). Cells were seeded in flasks containing supplemented media and were maintained in an incubator at 37 °C and 5% CO₂. 1:4 passes were performed twice per week. For the experiments, cells were seeded in 24 well-plates at a density of 2x10⁵ cells/well, or in 96 well-plates at a density of 8x10⁴ cells/well. For cytotoxicity experiments, cells were treated with the compounds before confluence with DMEM media supplemented with fetal bovine serum 1 %.

### Measurement of cellular viability: 3-(4,5-Dimethylthiazol-2-yl)-2,5-diphenyltetrazolium bromide, MTT

In order to assess cellular viability, we measured the metabolic reduction of 3-(4,5-Dimethylthiazol-2-yl)-2,5 diphenyltetrazolium bromide (MTT). MTT is an indirect assay to measure cellular viability. It is a colorimetric method based on the reduction of 3-(4,5-Dimethylthiazol-2-yl)-2,5 diphenyltetrazolium. This process is performed by the mitochondrial enzyme succinate-dehydrogenase present in metabolic active cells. Thus, the water-soluble yellow MTT (tetrazolium salt) is transformed into a insoluble purple compound (formazan). Quantity of living cells is directly proportional to the quantity of formazan (Mosmann, 1983, J Immunol Methods, 65: 55-63). Apoptotic cells present damaged mitochondria and do not perform the process. Therefore, with this assay we can measure cellular surviving and proliferation and also, we can determine the cytotoxicity of potential neuroprotective compounds. In order to assess cellular viability in SH-SY5Y cell line, we added 30 µl/well of MTT (5 mg/mL) and after 2 h, medium was carefully removed to avoid losing the formazan and was diluted in 300 µL of DMSO. Afterwards, samples were transferred to a 96 well-plate and the absorbance was measured at 570 nm using a absorbance/fluorescence reader FluoStar optima®. Every MTT assay was performed in triplicates. Absorbance values obtained with the toxic alone were subtracted to the absorbance values obtained in basal conditions (without treatment). That result was considered as the 100 % of cell death and the values obtained with the compounds in the presence of the toxic stimulus were normalized as percentages of that value. For determining the surviving percentage, those percentages of cell death were subtracted to 100.

Neuroprotection elicited by the compounds of the invention **1-16**: The activation of Nrf2-ARE pathway by different compounds exerts neuroprotective effects against toxic models based on oxidative stress via the expression of cytoprotective genes. Hence, we studied the neuroprotective properties of the compounds of this invention in different *in vitro* models of cytotoxicity induced by oxidative stress. We evaluated the neuroprotective potential of the compounds using the human neuroblastoma cell line using the following cytotoxicity models: 1) oxidative stress induced by rotenone + oligomycin, both blockers of the electron transport chain as previously described (Halliwell, 1992, J Neurochem, 59: 1609-23, Newhouse y col., 2004, Toxicol Sci, 79: 137-46), and 2) cytotoxicity induced by the electrophilic compound Menadione. This compound produces oxidative stress after being metabolized by microsomal enzymes in the cell which produces hydroxyl radical and oxygen reactive species. As a consequence, there is a depolarization of mitochondrial membrane generating a release of apoptotic factors such as cytochrome c, and activating the caspase cascade (Loor y col., 2010, Free Radic Biol Med, 49: 1925-36, Chuang y col., 2002, Cancer Res, 62: 6246-54). Caspase 3 activation can produce the lysis of proteins and induce DNA fragmentation, leading to cell death (Gerasimenko y col., 2002, J Cell Sci, 115: 485-97).

### 1) Neuroprotection against oxidative stress induced by the combination of rotenone (30 µM) and oligomycin (10 µM):

### a. Example 19a: Pre-incubation model:

These set of experiments is designed to study in detail the potential effect of the compounds inducing Nrf2 during the pre-incubation period, in which toxic stimuli are not present and therefore, we eliminated the potential free radical scavenging effect that presumably also possess the compounds. For this study we propose a protocol in which the cells are pre-incubated with each of the derivatives at 1 µM for 24 h. After the pre-incubation period, the medium was removed and replaced by culture medium with the rotenone / oligomycin-A (30 µM and 10 µM, respectively) toxic mixture.

In all the experiments, a positive control was used with comparative purposes and to evaluate the usefulness of the method. Melatonin (1 µM) was selected as positive control, because it has demonstrated neuroprotective properties in different oxidative stress models, including the rotenone / oligomycin model.

The results obtained for the compounds named as compounds 1 to 16 are shown in Table 3 and are expressed as percentage of cell survival and percentage of neuroprotective effect.

**Table 3**

| **Percentage of neuronal protection afforded by the compounds of the invention and melatonin at 1 µM. Data are expressed as the mean ± S.E.M of at least three experiments in triplicate of three different batches of cells.** | | | |
|---|---|---|---|
| **Compound** | **R** | **Rot/Olig (30/10) Pre-incubation** | |
| | | **% Survival** | **% Protection** |
| **Basal** | | 100 | |
| **Rote/Olig** | | 58.09 ± 2.17 | |
| **Melatonin** | | 71.76 ± 2.81 | 31.58*** |
| **1** | Me | 70.38 ± 4.03 | 28.48 ** |
| **2** | Ph | 72.65 ± 3.59 | 34.66** |
| **3** | *p*-Me-Ph | 67.03 ± 3.21 | 20.49*** |
| **4** | *o*-MeO-Ph | 67.07 ± 2.61 | 19.04** |
| **5** | *m*-OMe-Ph | 70.17 ± 2.42 | 27.07*** |
| **6** | *p*-OMe-Ph | 69.97 ± 3.04 | 29.79*** |
| **7** | *p*-OH-Ph | 71.55 ± 4.92 | 32.47*** |
| **8** | *m*-MeO, p-OH-Ph | 73.95 ± 3.13 | 37.00*** |
| **9** | *p*-F-Ph | 67.41 ± 2.13 | 21.52*** |
| **10** | *p*-Br-Ph | 71.11 ± 2.61 | 30.20*** |
| **11** | *p*-Cl-Ph | 73.17 ± 3.12 | 33.53*** |
| **12** | *m*,*p*-di-Cl-Ph | 70.12 ± 4.35 | 25.28** |
| **13** | *o*-CF₃-Ph | 66.96 ± 3.01 | 22.09** |
| **14** | *m*-CF₃-Ph | 72.45 ± 2.92 | 34.90*** |
| **15** | *p*-CF₃-Ph | 73.36 ± 3.31 | 37.46*** |
| **16** | *p*-NO₂-Ph | 68.45 ± 2.88 | 23.00** |

### b. Example 19b: Pre- and co-incubation model:

In this model, a 24 h pre-incubation period was performed in presence of the compounds synthesized at a concentration of 1 µM. Thereafter, a 24 h co-incubation period was added, in presence of the compounds with rotenone / oligomycin A (30 µM / 10 µM) mixture. After 24h, cell viability was assessed by the MTT reduction method. This protocol provides information on all potential biological activities present in the structure under study.

**Table 4**

| **Percentage of neuronal protection afforded by the compounds of the invention and melatonin at 1 µM. Data are expressed as the mean ± S.E.M of at least three experiments in triplicate of three different batches of cells.** | | | |
|---|---|---|---|
| **Compound** | **R** | **Rot/Olig (30/10) Pre y coincubation** | |
| | | **% Survival** | **% Protection** |
| **Basal** | | 100.00 | |
| **Rote/Olig** | | 48.64 ± 2.54 | |
| **Melatonin** | | 68.27 ± 4.27 | 31.08*** |
| **1** | Me | 76.98 ± 1.30 | 28.76*** |
| **2** | Ph | 73.69 ± 4.65 | 47.99*** |
| **3** | *p*-Me-Ph | 76.40 ± 3.89 | 55.14*** |
| **4** | *o*-MeO-Ph | 69.90 ± 4.17 | 28.05* |
| **5** | *m*-OMe-Ph | 78.70 ± 4.37 | 57.58*** |
| **6** | *p*-OMe-Ph | 71.57 ± 3.94 | 44.08*** |
| **7** | *p*-OH-Ph | 79.67 ± 2.37 | 39.76*** |
| **8** | *m*-MeO, p-OH-Ph | 81.09 ± 1.71 | 41.73*** |
| **9** | *p*-F-Ph | 69.62 ± 2.63 | 40.46*** |
| **10** | *p*-Br-Ph | 70.40 ± 2.21 | 40.75*** |
| **11** | *p*-Cl-Ph | 70.15 ± 3.70 | 28.44** |
| **12** | *m*,*p*-di-Cl-Ph | 69.86 ± 3.47 | 27.82** |
| **13** | *o*-CF₃-Ph | 73.83 ± 4.55 | 50.21*** |
| **14** | *m*-CF₃-Ph | 72.56 ± 3.75 | 45.94*** |
| **15** | *p*-CF₃-Ph | 72.96 ± 2.99 | 47.61*** |
| **16** | *p*-NO₂-Ph | 76.52 ± 2.18 | 29.53*** |

As the compound is present during the 24 hours prior to the incubation of the toxic stimuli, it is able to show its ability to induce Nrf2, thus, promoting cell survival. In addition, it is also present during the exposure to the toxic stimulus combination, the rotenone / oligomycin A mixture that causes the aberrant production of free radical species inside the cell. These radical species damage the cell and finally, induce cellular apoptosis. Besides Nrf2 induction, as derivatives are present during toxic stimuli period, they may further exhibit their free radical scavenger ability. Survival and protection percentages are summarized in Table 4.

### 2) Example 19c: Neuroprotection against oxidative stress induced by menadione:

To further confirm the neuroprotective capacity in another model of oxidative stress, menadione was selected as toxic stimulus. This compound is a polycyclic aromatic ketone (1,4-naphthoquinone) that has the ability to generate intracellular free radicals at multiple sites through chain redox radical reactions by the activation of NADPH / quinone oxidase. It is also capable of inducing DNA fragmentation and depleting the cell of the natural antioxidant glutathione. Further, it induces apoptosis through the down-regulation of ERK and JNK, followed by caspase 3 activation and ADP-ribose polymerase (PARP) disruption.

The studies of neuroprotection against menadione have been designed following the just mentioned pre- and co-incubation protocol. Human SH-SY5Y neuroblastoma cells were pre-incubated with each of the compounds at 1 µM for 24 h, after which the medium was replaced with fresh medium containing 8 µM of menadione, in presence or absence of the compounds (1 µM) for a further 24 h. Finally, cell viability was measured by the MTT reduction method.

**Table 5**

| **Percentage of neuronal protection afforded by the compounds of the invention and melatonin at 1 µM. Data are expressed as the mean ± S.E.M of at least three experiments in triplicate, of three different batches of cells.** | | | |
|---|---|---|---|
| **Compound** | **R** | **Menadione (8 mM) pre y co Sh-SY5Y** | |
| | | **% Survival** | **% Protection** |
| **Basal** | | 100 | |
| **Menadione** | | 62.49 ± 2.65 | |
| **Melatonin** | | 72.86 ± 5.24 | 37.35*** |
| **1** | Me | 87.65 ± 1.74 | 63.28*** |
| **2** | Ph | 73.99 ± 3.18 | 32.45*** |
| **3** | *p*-Me-Ph | 71.42 ± 2.43 | 25.01 * |
| **4** | *o*-MeO-Ph | 90.53 ± 1.97 | 67.12*** |
| **5** | *m*-OMe-Ph | 75.96 ± 3.31 | 38.11*** |
| **6** | *p*-OMe-Ph | 74.67 ± 2.91 | 35.77*** |
| **7** | *p*-OH-Ph | 86.76 ± 3.24 | 61.75*** |
| **8** | *m*-MeO, p-OH-Ph | 89.88 ± 1.86 | 69.39*** |
| **9** | *p*-F-Ph | 71.62 ± 3.74 | 29.65* |
| **10** | *p*-Br-Ph | 72.38 ± 3.75 | 27.62** |
| **11** | *p*-Cl-Ph | 85.65 ± 2.93 | 57.01*** |
| **12** | *m*,*p*-di-Cl-Ph | 86.53 ± 3.04 | 59.33*** |
| **13** | *o*-CF₃-Ph | 74.90 ± 2.19 | 36.22*** |
| **14** | *m*-CF₃-Ph | 78.46 ± 3.03 | 44.79*** |
| **15** | *p*-CF₃-Ph | 73.23 ± 3.43 | 31.64** |
| **16** | *p*-NO₂-Ph | 84.31 ± 2.15 | 53.57*** |

### Example 20

### Toxicoloigcal properties of the compounds object of this invention

In general, compounds capable of inducing the transcription factor Nrf2 may have a hepatotoxic effect, due to the electrophilic character that generally characterizes them. The derivatives objects of this invention were designed to avoid this toxicity and to improve its safety profile. The liver plays an essential role in the metabolism of endogenous and exogenous compounds. Hepatocytes capture these compounds and after metabolizing them, they are eliminated by different mechanisms. Metabolized compounds usually have less pharmacological activity and less toxicity than the corresponding active principles. However, in certain cases, the active principles or their metabolites may be toxic to the hepatocytes. Hepatic toxicity is one of the leading causes of drug withdrawal from preclinical studies. The HepG2 cell line shows most of the genotypic and phenotypic characteristics of normal liver cells, and they perform the typical functions of human liver cells. In addition, these cells have been defined by the European Medicines Agency (EMA) as a model of hepatoxicity by measuring the induction of apoptosis and / or cell survival. Therefore, the safety profile of the compounds object of the present invention was evaluated in the HepG2 hepatic cancer cell line. HepG2 cells were cultured in T75 flask using EMEM as culture medium supplemented with non-essential amino acids, 10% fetal bovine serum, 50 units / mL penicillin and 50 µg / mL streptomycin (reagents from GIBCO, Madrid, Spain). Cultures were maintained in an incubator with an O₂ / CO₂ gas mixture (95/5%) at 37 °C with a humid atmosphere and two passes per week were made when they reached 90% of confluence. All experiments were performed on cells that were between passages 5 and 15. For the experiments, the cells were plated in 96-well plates at a density of 4 × 10⁴ cells / well in 200 µl of culture medium. After 24 h in culture, the medium was removed and replaced with 100 µL of the corresponding solution of the compounds of the invention in culture medium at the selected concentrations (1, 10, 30 and 100 µM). The compounds were studied in triplicate, including in each experiment 3 wells without treatment, which was considered as 100% of viability (baseline conditions). After 24 h of incubation with the treatments, cell viability was assessed by the MTT reduction method.

In order to further study its safety profile, the effect of the derivatives on neuronal cells used for neuroprotection experiments, the SH-SY5Y human neuroblastoma cells, was also evaluated. Maintenance and culture methods of this cell line have been described above. For toxicity experiments, SH-SY5Y cells were plated in 96-well plates at the density of 6x10 ⁴ cells / well. The experimental conditions were the same as those described for the toxicity study in HepG2 cells.

**Table 6**

| **LD₅₀ of the compounds of the invention, measured as the reduction of cell viability in the HepG2 cell line and in the SH-SY5Y neuronal cell line. Data are expressed as the mean ± S.E.M of at least 3 experiments in triplicate of 3 different batches of cells.** | | | |
|---|---|---|---|
| **Compound** | **R** | **SH-SY5Y LD₅₀ (µM)** | **HepG2 LD₅₀ (µM)** |
| **Melatonin** | | > 100 | > 100 |
| **1** | Me | > 100 | > 100 |
| **2** | Ph | >100 | > 100 |
| **3** | *p*-Me-Ph | 80 | > 100 |
| **4** | *o*-MeO-Ph | 64 | > 100 |
| **5** | *m*-OMe-Ph | 83 | > 100 |
| **6** | *p*-OMe-Ph | 60 | > 100 |
| **7** | *p*-OH-Ph | > 100 | > 100 |
| **8** | *m*-MeO, *p*-OH-Ph | > 100 | > 100 |
| **9** | *p*-F-Ph | >100 | > 100 |
| **10** | *p*-Br-Ph | 60 | > 100 |
| **11** | *p*-Cl-Ph | > 100 | > 100 |
| **12** | *m*,*p*-di-Cl-Ph | 51 | > 100 |
| **13** | *o*-CF₃-Ph | >100 | > 100 |
| **14** | *m*-CF₃-Ph | >100 | > 100 |
| **15** | *p*-CF₃-Ph | 70 | > 100 |
| **16** | *p*-NO₂-Ph | >100 | > 100 |

The results obtained are shown in Table 6 expressed as the lethal dose 50 (LD₅₀), or needed concentration to reduce to 50% the viability of basal conditions (without treatment), measured by the MTT reduction method. In general, all compounds showed low toxicity in the SH-SY5Y human neuroblastoma cell line. On the other hand, none of them showed toxicity in the HepG2 hepatic cancer cell line; so that the compounds object of this invention show low or inexistent hepatoxicity.

## Claims

1. A compound of formula (I) 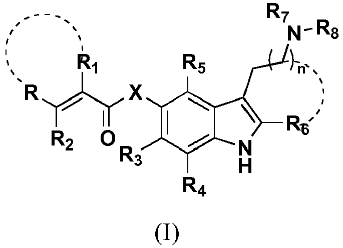 wherein
R is selected from the group consisting of:
- (C₁-C₆)alkyl optionally substituted by one, two, or three halogen atoms selected from fluorine, chlorine and bromine; (C₃-C₆)cycloalkyl; (C₁-C₆)alkoxyl; (C₃-C₆)cycloalkoxyl; cyano and nitro; or two groups can form together a group -O(CH₂)ₘO-, or -(CH₂)p-, or -CH=CH-CH=CH-; or
- phenyl optionally substituted by one, two or three groups independently selected form fluorine; chlorine; bromine; (C₁-C₆)alkyl optionally substituted by one, two, or three halogen atoms selected from fluorine, chlorine, and bromine; (C₃-C₆)cycloalkyl; (C₁-C₆)alkoxyl; (C₃-C₆)cycloalkoxyl; cyano and nitro; or two groups can form together a group -O(CH₂)ₘO-, -(CH₂)p-, or -CH=CH-CH=CH-; and/or
- an heteroaryl group selected from 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, 3-pyrazinyl, 2-pyrrolyl, 3-pyrrolyl, 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-imidazolyl, 4-imidazolyl, 3-pyrazolyl, 4-pyrazolyl, 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl, 1,3,4-oxadiazol-2-yl, 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl, 1,3,4-thiadiazol-2-yl, 1,2,3-triazol-4-yl, 1,2,4-triazol-3-yl and 5-tetrazolyl, being the heteroaryl group optionally substituted by one, two or three groups independently selected from fluorine, chlorine, and bromine, (C₁-C₆)alkyl, (C₃-C₆)cycloalkyl, (C₁-C₆)alkoxyl, (C₃-C₆)cycloalkoxyl, cyano and nitro;
R₁ and R₂ are selected from the group consisting of hydrogen, (C₁-C₆)alkyl, (C₃-C₆)cycloalkyl, and phenyl, optionally substituted by one, two or three groups independently selected from fluorine, chlorine, and bromine, (C₁-C₆)alkyl, (C₁-C₆)alkoxyl; (C₃-C₆)cycloalkyl, (C₃-C₆)cycloalkoxyl, cyano, nitro, and carboxilate or two groups can form together a group -O(CH₂)_{q}O-, -(CH₂)ᵣ-, or -CH=CH-CH=CH-;
R₃, R₄ and R₅ are selected from the group consisting of hydrogen (C₁-C₆)alkyl, (C₃-C₆)cycloalkyl, and phenyl, optionally substituted by one, two or three groups independently selected from fluorine, chlorine, and bromine, (C₁-C₆)alkyl, (C₁-C₆)alkoxyl; (C₃-C₆)cycloalkyl, (C₃-C₆)cycloalkoxyl, cyano, nitro, and carboxylate or two groups can form together a group -O(CH₂)_{q}O-, -(CH₂)ᵣ-, or -CH=CH-CH=CH-;
R₆ is selected from the group consisting of hydrogen, (C₁-C₆)alkyl, (C₃-C₆)cycloalkyl, and phenyl, optionally substituted by one, two or three groups independently selected form fluorine, chlorine, bromine, (C₁-C₆)alkyl, (C₁-C₆)alkoxyl; (C₃-C₆)cycloalkyl, (C₃-C₆)cycloalkoxyl, cyano and nitro;
R₇ is selected from the group consisting of hydrogen, (C₁-C₆)alkyl, (C₃-C₆)cycloalkyl, acetyl or phenyl, optionally substituted by one, two or three groups independently selected from fluorine, chlorine and bromine, (C₁-C₆)alkyl, (C₁-C₆)alkoxyl; (C₃-C₆)cycloalkyl, (C₃-C₆)cycloalkoxyl, cyano, nitro or two groups can form together a group -(CH₂)ₛ-, or -CH=CH-CH=CH-
R₈ is selected from the group consisting of hydrogen, (C₁-C₆)alkyl, (C₃-C₆)cycloalkyl, acetyl or phenyl, optionally substituted by one, two or three groups independently selected from fluorine, chlorine, and bromine, (C₁-C₆)alkyl, (C₁-C₆)alkoxyl; (C₃-C₆)cycloalkyl, (C₃-C₆)cycloalkoxyl; cyano and nitro or two groups can form together a group -(CH₂)ₛ-, or -CH=CH-CH=CH-
or R₇ = R₈ of formula =C=S
X is selected from an oxygen atom, a nitrogen atom or a sulphur atom, -SO- or SO₂
n is an integer selected from 0, 1, 2, 3, 4 or 5;
their salts, prodrugs, or solvates.

2. A compound according to claim 1, wherein
R is selected from the group consisting of phenyl optionally substituted by one or two groups independently selected from fluorine; chlorine; (C₁-C₆)alkyl optionally substituted by one, two, or three halogen atoms selected from fluorine, chlorine, and bromine; (C₁-C₆)alkoxyl and nitro; or one heterocycle optionally substituted by one or two groups independently selected from fluorine; chlorine; (C₁-C₆)alkyl; alkoxyl and nito,
R₁ is hydrogen;
R₂ is hydrogen;
R₃ is hydrogen;
R₄ is hydrogen;
R₅ is hydrogen;
R₆ is hydrogen;
R₇ is acyl (C2)
R₈ is hydrogen; and
n is an integer selected from 0, 1 and 2; and
their salts, prodrugs or solvates, preferably, their pharmaceutically acceptable salts.

3. A compound according to claim 1, wherein
R is phenyl optionally substituted by a group selected from fluorine; (C₁-C₆)alkyl optionally substituted by one, two, or three halogen atoms selected from fluorine, chlorine and bromine; (C₁-C₆)alkoxyl and nitro;
R₇ is acyl;
R₈ is hydrogen; and
n is 1; and
their salts, prodrugs or solvates, preferably, their pharmaceutically acceptable salts.

4. A compound according to claim 1, wherein
R is phenyl optionally substituted by a group selected from fluorine; (C₁-C₆)alkyl optionally substituted by one, two or three halogen atoms selected from fluorine, chlorine or bromine; (C₁-C₆)alkoxyl and nitro;
R₇ = R₈ is =C=S; and
n is 1; and
their salts, prodrugs or solvates, preferably, their pharmaceutically acceptable salts.

5. A compound according to claim 3, selected from:
• 3-(2-acetamidoethyl)-1*H*-indol-5-yl cinnamate
• 3-(2-acetamidoethyl)-1*H*-indol-5-yl (*E*)-3-(3-methoxyphenyl)acrylate
• 3-(2-acetamidoethyl)-1*H*-indol-5-yl (*E*)-3-(4-methoxyphenyl)acrylate
• 3-(2-acetamidoethyl)-1*H*-indol-5-yl (*E*)-3-(2-methoxyphenyl)acrylate
• 3-(2-acetamidoethyl)-1*H*-indol-5-yl (*E*)-3-(4-chlorophenyl)acrylate
• 3-(2-acetamidoethyl)-1*H*-indol-5-yl (*E*)-3-(4-bromophenyl)acrylate
• 3-(2-acetamidoethyl)-1*H*-indol-5-yl (*E*)-3-(4-fluorophenyl)acrylate
• 3-(2-acetamidoethyl)-1*H*-indol-5-yl (*E*)-3-(3-(trifluoromethyl)phenyl)acrylate
• 3-(2-acetamidoethyl)-1*H*-indol-5-yl (*E*)-3-(4-(trifluoromethyl)phenyl)acrylate
• 3-(2-acetamidoethyl)-1*H*-indol-5-yl (*E*)-3-(2-(trifluoromethyl)phenyl)acrylate
• 3-(2-acetamidoethyl)-1*H*-indol-5-yl (*E*)-3-(4-methylphenyl)acrylate
• 3-(2-acetamidoethyl)-1*H* indol-5-yl (*E*)-3-(3,4-dichlorophenyl)acrylate
• 3-(2-acetamidoethyl)-1*H*-indol-5-yl (*E*)-3-(4-nitrophenyl)acrylate
• 3-(2-acetamidoethyl)-1*H*-indol-5-yl (*E*)-2-butenoate
• 3-(2-acetamidoethyl)-1*H*-indol-5-yl (*E*)-3-(4-hydroxyphenyl)acrylate
• 3-(2-acetamidoethyl)-1*H*-indol-5-yl (*E*)-3-(4-hydroxy-3-methoxyphenyl)acrylate

6. A process for the preparation of a compound of formula (I) 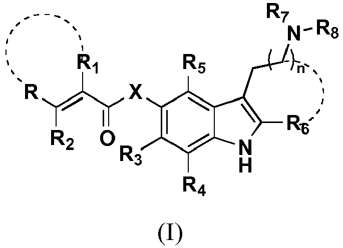 wherein
R is selected from the group consisting of:
- (C₁-C₆)alkyl optionally substituted by one, two, or three halogen atoms selected from fluorine, chlorine, and bromine; (C₃-C₆)cycloalkyl; (C₁-C₆)alkoxyl; (C₃-C₆)cycloalkoxyl; cyano and nitro; or two groups can form together a group -O(CH₂)ₘO-, or -(CH₂)p-, or -CH=CH-CH=CH-; or
- phenyl optionally substituted by one, two or three groups independently selected form fluorine; chlorine; bromine; (C₁-C₆)alkyl optionally substituted by one, two, or three halogen atoms selected from fluorine, chlorine, and bromine; (C₃-C₆)cycloalkyl; (C₁-C₆)alkoxyl; (C₃-C₆)cycloalkoxyl; cyano and nitro; or two groups can form together a group -O(CH₂)ₘO-, -(CH₂)p-, or -CH=CH-CH=CH-; and/or
- an heteroaryl group selected from 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, 3-pyrazinyl, 2-pyrrolyl, 3-pyrrolyl, 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-imidazolyl, 4-imidazolyl, 3-pyrazolyl, 4-pyrazolyl, 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl, 1,3,4-oxadiazol-2-yl, 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl, 1,3,4-thiadiazol-2-yl, 1,2,3-triazol-4-yl, 1,2,4-triazol-3-yl, and 5-tetrazolyl, being the heteroaryl group optionally substituted by one, two or three groups independently selected from fluorine, chlorine, and bromine, (C₁-C₆)alkyl, (C₃-C₆)cycloalkyl, (C₁-C₆)alkoxyl, (C₃-C₆)cycloalkoxyl, cyano and nitro;
R₁ and R₂ are selected from the group consisting of hydrogen, (C₁-C₆)alkyl, (C₃-C₆)cycloalkyl, and phenyl, optionally substituted by one, two, or three groups independently selected from fluorine, chlorine, and bromine, (C₁-C₆)alkyl, (C₁-C₆)alkoxyl; (C₃-C₆)cycloalkyl, (C₃-C₆)cycloalkoxyl, cyano, nitro and carboxilate or two groups can form together a group -O(CH₂)_{q}O-, -(CH₂)ᵣ-, or -CH=CH-CH=CH-;
R₃, R₄ and R₅ are selected from the group consisting of hydrogen (C₁-C₆)alkyl, (C₃-C₆)cycloalkyl, and phenyl, optionally substituted by one, two or three groups independently selected from fluorine, chlorine and bromine, (C₁-C₆)alkyl,(C₁-C₆)alkoxyl; (C₃-C₆)cycloalkyl, (C₃-C₆)cycloalkoxyl, cyano, nitro and carboxilate or two groups can form together a group -O(CH₂)_{q}O-, -(CH₂)ᵣ-, or -CH=CH-CH=CH-;
R₆ is selected from the group consisting of hydrogen, (C₁-C₆)alkyl, (C₃-C₆)cycloalkyl and phenyl, optionally substituted by one, two or three groups independently selected form fluorine, chlorine, bromine, (C₁-C₆)alkyl, (C₁-C₆)alkoxyl; (C₃-C₆)cycloalkyl, (C₃-C₆)cycloalkoxyl, cyano and nitro;
R₇ is selected from the group consisting of hydrogen (C₁-C₆)alkyl, (C₃-C₆)cycloalkyl, acetyl or phenyl, optionally substituted by one, two or three groups independently selected from fluorine, chlorine and bromine, (C₁-C₆)alkyl,(C₁-C₆)alkoxyl; (C₃-C₆)cycloalkyl, (C₃-C₆)cycloalkoxyl, cyano, nitro or two groups can form together a group -(CH₂)ₛ-, or -CH=CH-CH=CH-;
R₈ is selected from the group consisting of hydrogen (C₁-C₆)alkyl, (C₃-C₆)cycloalkyl, acetyl or phenyl, optionally substituted by one, two or three groups independently selected from fluorine, chlorine and bromine, (C₁-C₆)alkyl,(C₁-C₆)alkoxyl; (C₃-C₆)cycloalkyl, (C₃-C₆)cycloalkoxyl, cyano and nitro or two groups can form together a group -(CH₂)ₛ-, or -CH=CH-CH=CH-;
or R₇ = R₈ of formula =C=S;
X is selected from an oxygen atom, a nitrogen atom or an sulphur atom, -SO- or SO₂
n is an integer selected from 0, 1, 2, 3, 4 or 5;
and their salts, prodrugs or solvates,
that comprises the reaction of an acid of formula (II): 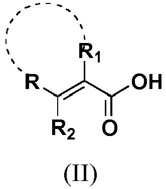 where R, R₁ and R₂ have the meaning previously indicated;
with a compound of formula (III): 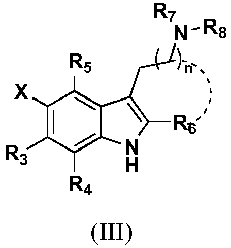 where, R₃, R₄, R₅, R₆, R₇, R₈ and n have the meaning previously indicated.

7. The process according to claim 6, wherein the reaction between the compound of formula (II) and the compound of formula (III) is performed in the presence of a catalyst selected from HATU, DCC, or EDCI, in a solvent selected from dichloromethane, 1,2-dichloroethane, chloroform and mixtures thereof

8. A pharmaceutical composition of a compound of formula (I) according to any one of claims 1 to 5, or a pharmaceutically acceptable salt, prodrug, or solvate thereof, and a pharmaceutically acceptable excipient.

9. Use of a compound of formula (I) according to any one of the claims 1 to 5, or a pharmaceutically acceptable salt, prodrug or solvate thereof, in the preparation of a pharmaceutical composition with Nrf2 induction effect and/or antioxidant and/or neuroprotectant and/or immunomodulator.

10. Use of a compound of formula (I) according to any one of claims 1 to 5, or a pharmaceutically acceptable salt, prodrug, or solvate thereof, in the preparation of a pharmaceutical composition for the prevention or the treatment of a central and/or peripheral neurodegenerative disease or of a cerebro-isquemic disease (ictus).

11. Use according to claim 9, wherein the neurodegenerative disease is Alzheimer's disease.

12. Use according to claim 9, wherein the neurodegenerative disease is Parkinson's disease.

13. Use according to claim 9, wherein the neurodegenerative disease is Huntington's disease.

14. Use according to claim 9, wherein the neurodegenerative disease is multiple sclerosis.

15. Use according to claim 9, wherein the neurodegenerative disease is cerebral ictus.

16. Use according to claim 9, wherein the neurodegenerative disease is amyotrophic lateral sclerosis.

17. Use according to any one of claims 8-16, wherein the compound of formula (I), or a pharmaceutically acceptable salt, prodrug, or solvate thereof, is used for the manufacture of a medicine for oral, parental, subcutaneous, intramuscular, intravenous, or rectal administration.

18. Use according to any one of claims 8 to 18, wherein the compound of formula (I), or a pharmaceutically acceptable salt, prodrug or solvate thereof, is administered in a daily dose between 0.1 and 100 mg/Kg of body weight.

19. Use according to claim 19, wherein the compound of formula (I), or a pharmaceutically acceptable salt, prodrug or solvate thereof, is administered in a daily dose between 2 and 5 mg/Kg of body weight.
